# EUROPEAN PATENT SPECIFICATION

(11) **EP 4 237 405 B1**
(45) Date of publication and mention of the grant of the patent: **02.12.2026**
(21) Application number: 21802226.7
(22) Date of filing: 26.10.2021
(51) Int. Cl.: C07C 213/10, A61K 8/41, C07C 217/76

(54) **2-METHOXYMETHYL-P-PHENYLENEDIAMINE IN COSMETIC GRADE QUALITY**
2-METHOXYMETHYL-P-PHENYLENDIAMIN IN KOSMETIKQUALITÄT
2-MÉTHOXYMÉTHYL-P-PHÉNYLÈNEDIAMINE DE QUALITÉ COSMÉTIQUE

(30) Priority: 27.10.2020 US 202063105981 P
(43) Date of publication of application: 06.09.2023
(73) Proprietor: Wella Germany GmbH, 64295 Darmstadt (DE)
(72) Inventor: SPECKBACHER, Markus, 84562 Mettenheim-Hart (DE); HARTMANN, Elmar, 36132 Eiterfeld (DE); SCHLOTZHAUER, Gerd, 36266 Heringen (DE); OSAN, Armin, 36179 Bebra (DE); ABEL, Heike, 36132 Eiterfeld (DE); SCHRAN, Andreas, 64295 Darmstadt (DE); HELBIG, Christian, 36414 Unterbreizbach (DE)
(74) Representative: Zimmermann & Partner Patentanwälte mbB
(86) International application number: PCT/EP2021/079696
(87) International publication number: WO 2022/090235

(56) References cited:
- WO-A1-2012/044758
- CN-B- 104 744 272

## Description

### FIELD OF THE INVENTION

The present invention relates to 2-methoxymethyl-p-phenylenediamine according to formula (1) below, or salts thereof, in cosmetic grade quality. This compound is known to the industry as low sensitizing major dye precursor used in oxidative hair dye compositions as replacement for traditional p-phenylenediamine or p-toluenediamine dye precursors.

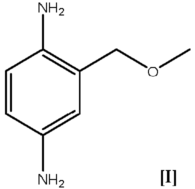

### BACKGROUND OF THE INVENTION

p-phenylenediamine derivatives are key precursors for oxidative hair dyeing, which have been used for decades for hair dyeing. The p-phenylenediamine derivatives are usually used to generate dark shades. Among the p-phenylenediamine derivatives, a particularly favourable candidate, namely 2-methoxymethyl-p-phenylenediamine has been identified. This dye precursor is particularly advantageous in that it is typically characterized by a lower sensitizing potential than traditional p-phenylenediamine or p-toluenediamine dye precursors.

Synthetic routes for manufacturing 2- methoxymethyl-p-phenylenediamine or salts thereof are known in the art.

For example, US2003/0041392A1 discloses a process for the preparation of 2-methoxymethyl-p-phenylenediamine via a Smiles rearrangement in one of the intermediate steps. Disadvantages of the process are harsh reaction conditions and the use of reactants such as trioxane (formaldehyde trimer) which may create health hazards for the workers in the production line. In addition, the process produces large amounts of waste solvent solutions containing sulfuric acid or toluene. These solutions cannot be recycled for the process but have to be discarded. Yields for the process according to US2003/0041392A1 are in the order of 50% of theoretical.

Another feasible synthetic route is disclosed in WO2012044758A1, which synthetic route comprises a combination of steps starting with 2-chlorobenzylchloride and methanol to form the methoxymethyl intermediate. Nitration occurs in 4 position and activates the chloride as leaving group. Substitution of the chloride by an amino donor, preferably using benzylamine, requires a phase-transfer catalyst to obtain the aniline intermediate. Final hydrogenation leads to the desired 2-methoxymethyl-p-phenylenediamine. Disadvantages of this method include harsh nitrosation conditions (using mixtures of sulfuric acid and fuming nitric acid), and an overall yield that may be relatively low. Furthermore, the carbon balance is insufficient, since the reactant benzylamine merely contributes a nitrogen atom, while the remainder of the molecule is discarded in the form of toluene-containing mixtures. A particular disadvantage of this method is that the product obtained comprises amorphous material which may lead to unwanted side effects such as surface oxidation. Surface oxidation in turn may negatively impact the appearance of the powdered material which can be a success criterion for cosmetic applications/formulations.

The present inventors recently found that 2-methoxymethyl-p-phenylenediamine produced by the known manufacturing methods bears the risk of deterioration, in that the appearance of the product obtained may change from a slightly to moderate or even significant dark coloration. These unwanted coloration effects are typically caused by surface oxidation as for example described above. Such surface oxidation often is observed after storage for several weeks or months, in particular at storage conditions involving temperatures exceeding 25°C and/or high humidity, for examples humidities exceeding 50%. In some cases, the surface oxidation may even be observed right after completing the synthesis, however. The observed impurities color the product 2-methoxymethyl-p-phenylenediamine from a beige/off-white appearance to a dark violet to greyish mass, and even to blackish coloration in different gradation. When used in hair coloring applications, this colored 2-methoxymethyl-p-phenylenediamine may have a negative impact on the desired color result on hair.

Once such a failure in appearance has been detected, the material does no longer qualify for being used in hair coloring applications. The customers expect the product to exhibit a cosmetically white or off-white appearance, and any dark colorations are perceived by customers as minor quality. If coloration forms on shelf at the customers' facilities, this is a most critical observation as this finding will lead to undesirable product re-calls which causes major disruptions in supply chains and has a strong negative impact on economic balances.

Therefore, a need exists to find out specification requirements for 2-methoxymethyl-*p-*phenylenediamine to be used in cosmetic applications, and in particular for hair dyeing. Furthermore, there exists a need for methods suitable for converting 2-methoxymethyl-p-phenylenediamine starting material having an impurity degree non-acceptable for cosmetic applications to 2-methoxymethyl-p-phenylenediamine meeting the specifications for cosmetic applications. In particular, a need exists for methods which allow for converting 2-methoxymethyl-*p*-phenylenediamine having colorations as described above to 2-methoxymethyl-2-phenylenediamine suitable for cosmetic applications with limited experimental effort, low cost and/or attractive timings. In regard of an increasing global demand of the said material, an economical access to a recrystallization process to even convert a whole production campaign into a material which again meets cosmetic specifications is highly desired. As indicated above, such recrystallization process shall be applicable to 2-methoxymethyl-2-phenylenediamine regardless of the product life cycle, that is it shall be applicable to freshly produced material where this coloration has been detected but also to material having developed such coloration during its shelf life, which might be even more important concerning the impact on various supply chains.

2-methoxymethyl-2-phenylenediamine is a material which is challenging to obtain in crystallized form, which may reduce the risk of unwanted coloration. Comparing the chemical structure of 2-methoxymethyl-p-phenylenediamine with p-phenylene-diamine and 2,5-toluene-diamine, the methoxymethyl group in 2-methoxymethyl-p-phenylenediamine is bulkier and makes it more difficult to form a regular crystal lattice.

One challenge addressed by the present invention is identifying for 2-methoxymethyl-p-phenylenediamine the requirements defining a cosmetic grade product quality with:
- an acceptable appearance and quality to be used in cosmetic applications without negative impacts, in particular for use in hair coloring applications
- a shelf-life of at least 9 months, in particular of at least 12 months under standard storage conditions (25°C or less and 45% humidity or less)

Another challenge addressed by the present invention is identifying specific methods and respective conditions for:
- isolating 2-methoxymethyl-p-phenylenediamine in cosmetic grade product quality, having such acceptable appearance and quality to be used in cosmetic applications
- stabilizing the product obtained in the cosmetic grade product quality for at least 9 months, in particular at least 12 months under standard storage conditions

Any stabilization of the product quality for at least 9 months, in particular at least 12 months would meet typical turn-around times for the usage of the material while being stored on shelf in real industrial scenarios.

CN104744272B discloses a preparation method for 2-methoxymethyl-p-phenylenediamine, which method encompasses as a final step the purification of the 2-methoxymethyl-p-phenylenediamine prepared in the previous steps.

### SUMMARY OF THE INVENTION

Subject matter of the present invention is a method as defined in claim 1 for assessing the quality of 2-methoxymethyl-p-phenylenediamine. Further subject matter of the present invention are methods as defined in claims 2 and 5, respectively, for converting colored 2-methoxymethyl-p-phenylenediamine starting material having an impurity degree non-acceptable for cosmetic applications to 2-methoxymethyl-p-phenylenediamine meeting the requirements for cosmetic applications. The dependent claims relate to particular embodiments thereof.

According to an aspect, subject matter of the present invention is a method for assessing the quality of 2-methoxymethyl-p-phenylenediamine. In particular, the method aims at assessing whether or not a given batch of 2-methoxymethyl-p-phenylenediamine exhibits cosmetic grade quality.

According to another aspect, subject matter of the present invention are methods for converting colored 2-methoxymethyl-p-phenylenediamine starting material having an impurity degree non-acceptable for cosmetic applications to 2-methoxymethyl-p-phenylenediamine meeting the requirements for cosmetic applications.

According to an embodiment, the present invention relates to a method for converting colored 2-methoxymethyl-p-phenylenediamine starting material having an impurity degree non-acceptable for cosmetic applications to 2-methoxymethyl-p-phenylenediamine meeting the requirements for cosmetic applications, the colored starting material being deteriorated by surface oxidation upon storage for several weeks or months. The method comprises the following steps:

| | |
|---|---|
| B | preparing a mixture of 100 parts-by-weight of the colored deteriorated 2-methoxymethyl-p-phenylenediamine starting material, activated charcoal, and a sufficient amount of aromatic solvent for dissolving the 2-methoxymethyl-p-phenylenediamine, |
| C | heating the mixture of step B to a first target temperature, |
| E | removing the activated charcoal by hot-filtering, thereby obtaining a filtrate, |
| F | cooling the filtrate to a second target temperature, |
| H | cold-filtering the filtrate, thereby obtaining a residue of recrystallized 2-methoxymethyl-p-phenylenediamine. |

When carrying out the method, all steps preferably are carried out in moisture-free conditions under an inert atmosphere in order to protect the 2-methoxymethyl-p-phenylenediamine from hydrolysis and oxidation. The aromatic solvent is selected from toluene, xylene, anisole, cresol, and combinations thereof.

According to another embodiment, the present invention relates to a method for converting colored 2-methoxymethyl-p-phenylenediamine starting material having an impurity degree non-acceptable for cosmetic applications to 2-methoxymethyl-p-phenylenediamine meeting the requirements for cosmetic applications, the colored starting material being deteriorated by surface oxidation upon storage for several weeks or months. The method comprises the following steps:

| | |
|---|---|
| K | preparing a mixture of 100 parts-by-weight of the colored deteriorated 2-methoxymethyl-p-phenylenediamine starting material, activated charcoal, and a sufficient amount of non-aromatic solvent for dissolving the 2-methoxymethyl-p-phenylenediamine, |
| L | heating the mixture of step K to a third target temperature, |
| N | removing the activated charcoal by filtering, thereby obtaining a filtrate, |
| P | bringing the filtrate to a fourth target temperature, and under reduced pressure reducing the amount of non-aromatic solvent in the filtrate by distillation, |
| Q | adding aromatic solvent to the filtrate, |
| R | distilling the filtrate under reduced pressure at the fourth target temperature until obtaining a ratio of 700 or less parts-by-volume of organic solvent per 100 parts-by-weight of the 2-methoxymethyl-p-phenylenediamine starting material, |
| S | increasing the pressure to ambient pressure, and cooling the filtrate to a fifth target temperature, |
| U | filtering the filtrate at the fifth target temperature, thereby obtaining a residue of recrystallized 2-methoxymethyl-p-phenylenediamine. |

When carrying out the method, all steps preferably are carried out in moisture-free conditions under an inert atmosphere in order to protect the 2-methoxymethyl-p-phenylenediamine from hydrolysis and oxidation. The non-aromatic solvent is selected from methanol, ethanol, iso-propanol, n-propanol, ethyl formiate, methyl acetate, ethyl acetate, methyl propionate, ethyl propionate, CHCl₃, CCl₄, THF, 1,4-dioxane, or combinations thereof. The aromatic solvent is selected from toluene, xylene, anisole, cresol, and combinations thereof.

The above methods may further comprise the step of determining that the colored 2-methoxymethyl-p-phenylenediamine starting material fails to exhibit cosmetic grade quality, and/or determining the quality of the final product. These determination steps may be carried out in water and in the presence of air oxygen. Excessive degradation during these determination steps may be avoided by the presence of antioxidant.

According to yet another aspect, the present invention relates to cosmetic grade 2-methoxymethyl-p-phenylenediamine. According to still another aspect, the present invention relates to 2-methoxymethyl-p-phenylenediamine with an acceptable shelf-life.

### DEFINITIONS

In the specification that follows , the term "2-methoxymethyl-p-phenylenediamine" may be abbreviated as "MBB".

As used in the specification and the appended claims, the singular forms "a," "an" and "the" include plural referents unless the context clearly dictates otherwise.

The term "may" in the context of this application means "is permitted to" or "is able to" and is a synonym for the term "can." The term "may" as used herein does not mean possibility or chance.

The term "and/or" in the context of this application means one or the other or both. For example, an aqueous solution of A and/or B means an aqueous solution of A alone, an aqueous solution of B alone and an aqueous solution of a combination of A and B.

The term "about" is understood to mean ±10 percent of the recited number, numbers or range of numbers. According to embodiments, the term "about" is understood to mean ±2 percent of the recited number, numbers or range of numbers.

The term "about 0% by weight" is understood to mean that no substance, compound or material to which zero (0) refers is present, up to a negligible but detectable amount is present, assuming that detectability is on a ppm (parts per million) basis.

Where features or aspects of the invention are described in terms of Markush groups, those skilled in the art will recognize that the invention is also thereby described in terms of any individual member or subgroup of members of the Markush group. For example, if X is described as selected from the group consisting of methyl, ethyl or propyl, claims for X being methyl and claims for X being methyl and ethyl are fully described. Moreover, where features or aspects of the invention are described in terms of Markush groups, those skilled in the art will recognize that the invention is also thereby described in terms of any combination of individual members or subgroups of members of Markush groups. Thus, for example, if X is described as selected from the group consisting of bromine, chlorine, and iodine, and Y is described as selected from the group consisting of methyl, ethyl, and propyl, claims for X being bromine and Y being methyl are fully described.

If a value of a variable that is necessarily an integer, e.g., the number of carbon atoms in an alkyl group or the number of substituents on a ring, is described as a range, e.g., 0-4, what is meant is that the value can be any integer between 0 and 4 inclusive, i.e., 0, 1, 2, 3, or 4. Similarly, values expressed in a range format should be interpreted in a flexible manner to include not only the numerical values explicitly recited as the limits of the range, but also to include all the individual numerical values or sub-ranges encompassed within that range as if each numerical value and sub-range were explicitly recited. For example, a range of "about 0.1% to about 5%" or "about 0.1% to 5%" should be interpreted to include not just about 0.1% to about 5%, but also the individual values (e.g., 1%, 2%, 3%, and 4%) and the sub-ranges (e.g., 0.1% to 0.5%, 1.1% to 2.2%, 3.3% to 4.4%) within the indicated range.

As used herein, the term "optionally" means that the corresponding step or feature may or may not be present. It includes both possibilities.

The terms "parts-by-weight" (PBW) and "parts-by-volume" (PBV) are used in their usual meaning. When used in a mixed context, for example an amount of a component indicated in PBW in admixture with a component indicated in PBV, the terms PBW and PBV as used herein denote the amount in grams of the component indicated in PBW relative to a volume in milliliters of the amount of the component indicated in PBV. For example, a mixture denoted herein as comprising 1 PBW 2-methoxymethyl-p-phenylenediamine in (or per) 5 PBV organic solvent comprises 1 gram 2-methoxymethyl-p-phenylenediamine per 5 ml organic solvent (or 1 mg per 5 microliter, or 1 kg per 5 liters, etc.). Assuming an approximated specific weight of the 2-methoxymethyl-p-phenylenediamine of 1.0 g/ml, the total volume of such 1:5 mixture would be 6 PBV.

The term "moisture-free conditions" as used herein is intended to mean the absence or exclusion of water deliberately added in pure form or in the form of an aqueous solution, or in the form of moisture/humidity. Reagent grade quality of the components used, in particular of the solvents used usually is sufficient, however, and specifically drying any the of components used in the recrystallization methods according to the present invention usually is not required but of course may be done. If desired, drying the solvents prior to use in the recrystallization methods according to the present invention may be accomplished by routine methods, for example by distillation or storage in the presence of desiccants. Drying the activated charcoal, if desired, may be accomplished for example by heating and subsequent storage under exclusion of moisture/humidity. Drying the inert gas, if desired, may be done by directing the gas flow through a bed of particulate desiccants. Since the major contributors to moisture are the solvents used in the recrystallization methods of the present invention, the term "moisture-free conditions" is defined as a water content of less than 2.5 percent, based on the weight of the solvent(s) used in the recrystallization method, in particular a water content of less than 1.0 percent, for example less than 0.5 percent.

The term "inert atmosphere" denotes the essential absence of components in the said atmosphere which might be reactive with components used in the recrystallization method of the present invention. In particular, the term "inert atmosphere" is intended to essentially exclude the absence of components reactive with MBB, such as oxygen. Suitable gases for providing an inert atmosphere include for example nitrogen, the noble gases and mixtures thereof. Considering the costs involved, nitrogen is a preferred inert gas.

The term "precipitation" and "crystallization" are used herein essentially interchangeably, and denote the transformation of MBB in solution to a solid form. It is noted that "precipitation" often is used in the chemical literature to denote a rapid transformation from solubilized to solid phase in an amorphous form, while "crystallization" mostly is used in the chemical literature to denote slow transformatiuon from solubilized to solid phase, thereby forming a crystal lattice or even large crystals. In the present specification and the appended claims, both terms are intended to denote at least a certain degree of crystallization. The formation of amorphous MBB according to the present invention is less preferred.

The term "MBB crystallization on-set temperature" denotes the temperature at which the concentration of MBB exceeds the solubility limit of MBB in a respective solution (in solvent with contaminants present) at the pressure involved. When cooling a respective solution, MBB starts to crystallize at the MBB crystallization on-set temperature (if not kinetically hindered).

The term "target temperature" as used herein defines a temperature range to be reached or held during a respective method step, or to be more precisely defines a temperature range in combination with a pressure range. As used herein, the "first", "second", "third" and "fifth" "target temperatures" define temperature ranges indicated in the respective contexts below at ambient pressure (1000 mbar ± 10% = 101.325 kPa ± 10%). The "fourth target temperature" defines the temperature range indicated in the respective context below at the reduced pressures indicated in the respective context below.

### DETAILED DESCRIPTION OF THE INVENTION

### 1 Quality grading of 2-methoxymethyl-p-phenylenediamine

According to an aspect, the present invention relates to assessing the quality of 2-methoxymethyl-p-phenylenediamine (MBB), and in particular the present invention relates to assessing whether a given MBB exhibits a quality sufficient for cosmetic applications.

The present inventors have found out that MBB as produced may comprise impurities, or may deteriorate during its shelf-life. The presence of such impirities, or the formation thereof appears to inter-related with the grade of crystallization of the MBB. It was noted that a high grade of defined crystals forming a regular crystal lattice provides for protection of MBB against such degradation. Impurities already initially present of formed over the time result in a coloration and may have negative impacts on the suitability of the product for cosmetic applications, in particular for hair dyeing applications. These impurities are believed to be the result of degradation or oxidation processes promoted by, inter alia:
- the presence of amorphous material, or generally a low degree of crystallization
- moisture/humidity, in particular humidities exceeding 50%
- elevated temperatures, in particular temperatures exceeding 28°C
- an oxygen-containing atmosphere
- daylight, and in particular UV radiation

The present inventors found out that the quality of MBB can be assessed by determining UV absorbance thereof. In particular, the present inventors found out that the quality grade of MBB can be assessed in terms of its maximum UV absorbance specifically at 525 nm.

Defining "cosmetic grade quality" of MBB in terms of its UV absorbance at 525 nm is sufficient for the purposes of the present invention. Other specifications of MBB in cosmetic grade quality comprise:
- purity: at least 99.0 area% at 254 nm, as determined by HPLC
- MBB assay: at least 98.0 area%, as determined by HPLC, or at least 98.0 % w/w, as determined by quantitative NMR
- residual solvent content: 1000 ppm or less, as determined by HPLC
- residual water content: 0.1% w/w or less, as determined by Karl-Fischer-Titration

One aspect of the present invention is a method for assessing the quality of 2-methoxymethyl-p-phenylenediamine. In particular, the method aims at assessing whether or not a given MBB exhibits cosmetic grade quality. The method comprises:
i determining at 525 nm the UV absorbance of an aqueous solution of 2-methoxymethyl-p-phenylenediamine,
ii grading the quality of the 2-methoxymethyl-p-phenylenediamine on basis of the UV absorbance obtained.

For the purposes of the present invention, the UV absorbance is determined in aqueous solution. The measurement is carried out under standard conditions (ambient temperature and pressure) using a 10 mm pathway (cuvette). The concentration of the MBB is 1.0 PBW in a total volume of 100.0 PBV distilled water. In order to avoid excessive degradation of the MBB during the measurement, the MBB desirably is protected by the presence of an antioxidant in the solution. Suitable antioxidants include for example ascorbic acid and sulfites, such as sodium sulfite. Suitable amounts of antioxidant typically are within the range 25-50 parts-by-weight per 100 parts-by-weight MBB starting material. A solution to be used in the determination of the UV absorbance of MBB typically is prepared by dissolving the antioxidant first, and subsequently adding the required amount of MBB to the solution. A solution for determining the UV absorbance may be prepared, for example, by dissolving 0.30 g ascorbic acid in 80 ml distilled water in a 100 ml volumetric flask, adding 1.0 g MBB, completely dissolving the MBB, for example by sonication, and filling up with distilled water to 100 ml. The measurement then is carried out at 525 nm under standard conditions using a 10 mm pathway (cuvette).

According to the present invention, the UV absorbance is determined at 525 nm. Through intensive testing, the present inventors found out that in order to be suitable for cosmetic applications, in particular for hair dyeing applications, MBB is required to exhibit a UV absorbance at 525 nm of less than 0.0080, preferably a UV absorbance of 0.0078 or less. MBB having an UV absorbance at 525 nm above 0.0082 was found to be non-acceptable for cosmetic applications. MBB having an UV absorbance at 525 nm between 0.0080 and 0.0082 only exceptionally should be used in case of acute shortage, and if the products prepared with such low quality MBB are intended for short-term use, i.e. will not be stored for more than one month.

### 2 Recrystallizing 2-methoxymethyl-p-phenylenediamine to cosmetic grade quality

According to an aspect, the present invention relates to methods for converting colored 2-methoxymethyl-p-phenylenediamine starting material having an impurity degree non-acceptable for cosmetic applications to 2-methoxymethyl-p-phenylenediamine meeting the requirements for cosmetic applications.

### 2.1 Recrystallization method allowing for complete recycling of the solvent used

According to the present invention, a method for converting colored 2-methoxymethyl-p-phenylenediamine starting material having an impurity degree non-acceptable for cosmetic applications to 2-methoxymethyl-p-phenylenediamine meeting the requirements for cosmetic applications, the colored starting material being deteriorated by surface oxidation upon storage for several weeks or months, comprises:

| | |
|---|---|
| B | preparing a mixture of 100 parts-by-weight of the colored deteriorated 2-methoxymethyl-p-phenylenediamine starting material, activated charcoal, and a sufficient amount of aromatic solvent for dissolving the 2-methoxymethyl-p-phenylenediamine, |
| C | heating the mixture of step B to a first target temperature, |
| E | removing the activated charcoal by hot-filtering, thereby obtaining a filtrate, |
| F | cooling the filtrate to a second target temperature, |
| H | cold-filtering the filtrate, thereby obtaining a residue of recrystallized 2-methoxymethyl-p-phenylenediamine. |

The aromatic solvent used in the recrystallization method is selected from toluene, xylene, anisole, cresol, and combinations thereof. Toluene is the presently preferred aromatic solvent.

In order to protect MBB from oxidation, all of the above steps preferably are carried out in moisture-free conditions under an inert atmosphere. In other words, access of moisture and air oxygen to the reaction vessel and the end product obtained preferably is limited in order to reduce yield loss. Excluding oxygen and moisture/humidity suitably increases the yield of MBB. It is usually preferable to maintain moisture-free conditions and the presence of an inert atmosphere as much as possible.

A sufficient amount of the aromatic solvent for dissolving the colored MBB is used in step B of the recrystallization method. A sufficient amount of the aromatic solvent is within the range of 500-900 parts-by-volume per 100 parts-by-weight MBB, typically within the range of 600-800 parts-by-volume. More typically, the amount is within the range of 650-750 parts-by-volume, in particular 680-720 parts-by-volume. For example, the amount of the aromatic solvent may be about 700 parts-by-volume per 100 parts-by-weight MBB, in particular if the aromatic solvent is toluene.

The mixture in step B is conveniently prepared by first dissolving the colored MBB starting material in the aromatic solvent, typically at ambient temperature (20-25°C) and ambient pressure, and subsequently adding the solution to the activated charcoal previously placed in the reaction vessel used for the recrystallization method, or vice versa. Alternatively, solvent, MBB and activated charcoal may be added essentially simultaneously to the reaction vessel used in the recrystallization method. Prior to adding MBB, the reaction vessel is preferably purged with inert gas.

The amount of activated charcoal used in step B typically is within the range of 3-20 parts-by-weight per 100 parts-by-weight MBB. The exact amount may be adjusted depending on the extent of coloration of the MBB starting material, or contamination level, respectively. For most contamination levels, 5-15 and for example 5-12, such as 5-10 parts-by-weight activated charcoal per 100 parts-by-weight MBB should be sufficient. Less than 3 parts-by-weight activated charcoal per 100 parts-by-weight MBB may not be sufficient for decreasing the levels of contaminants to the extent desired (or if it is sufficient, the time and effort for conducting the recrystallization method may not be justifed). Amounts of activated charcoal exceeding 20 parts-by-weight per 100 parts-by-weight MBB usually will not be required, except maybe for extremely high contamination levels. Since the activated charcoal adds to the expenses of the method, the amount actually added should be balanced with the actual level of contaminations.

The type of activated charcoal used in not specifically critical, and activated charcoals commercially available have been found suitable.

The mixture prepared in step B preferably is held in moisture-free conditions under an inert atmosphere. As such, the reaction vessel comprising the said mixture preferably is integrated into a closed system connected to a vacuum source and a supply of inert gas, which system is adapted to enable conducting preferably all steps of the recrystallization method in moisture-free conditions under an inert atmosphere.

The mixture of charcoal and MBB/solvent solution is heated in step C of the recrystallization method to a first target temperature. Suitably, the mixture is stirred while being heated to the first target temperature. It is understood that the heating rate is not particularly critical. Heating rates of 0.5-1.0°C per minute have been found suitable for heating the mixture of MBB, solvent and activated charcoal to the first target temperature. Heating rates below or above this range may be used, however, provided that the specific heating rate does not entail specific disadvantages such as for example an increased decomposition of the MBB.

The mixture of charcoal and MBB/solvent solution is heated in step C of the recrystallization method to a first target temperature. In the concentrations contemplated, the MBB is completely soluble in the aromatic solvent at the first target temperature and at ambient pressure. In other words, the first target temperature corresponds to the MBB crystallization on-set temperature or is a temperature higher than the MBB crystallization on-set temperature. Typically, the first target temperature is a temperature 10°C or more, suitably 15°C or more, and sometimes 20°C or more above the MBB crystallization on-set temperature. Suitably, the first target temperature is within the range of 60-100°C. According to embodiments, the first target temperature may be within the range of 70-90°C, in particular within the range of 75-85°C. According to specific embodiments, the first target temperature may be within the range of 78-82°C. For example, the first target temperature may be about 80°C. Step C preferably is carried out in moisture-free conditions under an inert atmosphere.

When the mixture has been brought to the first target temperature, the activated charcoal is removed by filtering. Prior to filtering, the method further may comprise optional step D:

| | |
|---|---|
| D | maintaining the mixture at the first target temperature for a time sufficient to at least partially adsorb dissolved impurities on the activated charcoal. |

By prolonging the duration of the exposure to the first target temperature in the presence of the activated charcoal, optional step D assists in absorbing more contaminants present in the mixture on the surface of the activated charcoal. Suitably, the mixture is stirred while being maintained at the first target temperature. According to embodiments, the mixture is maintained in step D at the first target temperature for 8-30 minutes. Typically, maintaing the mixture in step D for 8-15 minutes will be sufficient in order to achieve significant adsorption of contaminants present. Conveniently, the mixture may be held at the first target temperature in step D for 9-11 minutes, for example about 10 minutes. Optional step D preferably is carried out in moisture-free conditions under an inert atmosphere.

In step E of the recrystallization method, the activated charcoal is removed by hot-filtering. The filtering is carried out at a temperature above the MBB crystallization on-set temperature, in order to reduce yield loss due to the filtration step. According to embodiments, said hot-filtering in step E is done at the first target temperature, for example within the range of 60-100°C. According to embodiments, the hot-filtering in step E may be done at a temperature within the range of 70-90°C, in particular within the range of 75-85°C. According to specific embodiments, the hot-filtering in step E may be done at a temperature within the range of 78-82°C. For example, the hot-filtering in step E may be done at a temperature of about 80°C.

The activated charcoal filtered-off in step E may be discarded. The filtrate obtained after having removed the activated charcoal comprises the solution of MBB in the aromatic solvent, with a reduced level or amount of contaminants as compared to the MBB starting material.

In order to further reduce yield loss due to the filtration step, filtration step E optionally may comprise washing the filtered-off activated charcoal. According to embodiments, filtration step E optionally may comprise the indicated steps in numerical order:

| | |
|---|---|
| E1 | removing the activated charcoal by hot-filtering, |
| E2 | washing the filter residue with said aromatic solvent, and obtaining the washing liquid by hot-filtering, |
| E3 | combining the washing liquid with the filtrate. |

The washing liquid used in optional step E2 exhibits a temperature above the MBB crystallization on-set temperature, and in particular may exhibit a temperature within the range of the first target temperature.

According to embodiments, the filter residue is washed in step E2 up to three times each with 0.2-1.0 parts-by-volume, in particular 0.4-0.6 parts-by-volume. For example, the filter residue may be washed in step E2 twice or three times each with about 0.5 parts-by-volume of the aromatic solvent. According to embodiments, the filter residue may be washed in step E2 with a total of about 1.0 parts-by-volume of the aromatic solvent. The washing liquid is filtered off by hot-filtering as described for step E, collected and combined with the filtrate.

Filtration step E, and optional steps E1-E3 preferably is/are carried out in moisture-free conditions under an inert atmosphere.

After having removed the activated charcoal in filtration step E, the filtrate is cooled in step F to a second target temperature. Suitably, the filtrate is stirred while being cooled to the second target temperature. Cooling step F preferably is carried out in moisture-free conditions under an inert atmosphere. Cooling the filtrate may comprise active cooling, or allowing the filtrate to cool without active cooling.

It has been found out that the initial cooling phase from a higher temperature such as the first target temperature to the MBB crystallization on-set temperature is not particularly critical. For example, the filtrate may be cooled during the initial cooling phase to the MBB crystallization on-set temperature at a cooling rate of 1.0-2.0°C per minute. At the MBB concentrations and type of solvents contemplated and at ambient pressure, the MBB crystallization on-set temperature typically is within the range of about 55-60°C. When using toluene as the solvent, for example, the MBB crystallization on-set temperature is about 55°C. Cooling rates below or above this range may be used, however, provided that the specific cooling rate does not entail specific disadvantages, such as for example premature precipitation of MBB.

For the cooling phase from the MBB crystallization on-set temperature to the second target temperature, the cooling rate is preferably controlled in order to allow for an appropriate crystallization of the MBB, essentially avoiding uncontrolled precipitation. Cooling rates of 0.5-2.0°C per minute have been found suitable for cooling the filtrate from the MBB crystallization on-set temperature to the second target temperature. According to embodiments, the cooling rate from the MBB crystallization on-set temperature to the second target temperature is 0.5-1.0°C per minute.

As the filtrate is cooled from the MBB crystallization on-set temperature to the second target temperature, the initial clear filtrate turns into a suspension due to the formations of MBB crystals. Any crystals sticking to the wall of the reaction vessel may be removed mechanically by scraping-off using appropriate devices of the system.

The second target temperature is a temperature at which the solubiliy of MBB in the solvent is sufficiently low in order to keep low potential yield loss in the subsequent filtration step. A suitable second target temperature is less than 8.0°C. According to embodiments, the second target temperature may be within the range of 0.0-5.0°C, in particular within the range of 0.0-4.0°C. According to specific embodiments, the second target temperature may be within the range of 0.0-3.0°C. For example, the second target temperature may be about 3.0°C.

When the filtrate has been brought to the second target temperature, the MBB is collected as a solid residue by filtering, thereby removing the solvent. Prior to filtering, the method further may comprise optional step G:

| | |
|---|---|
| G | maintaining the filtrate at the second target temperature to allow for precipitation of 2-methoxymethyl-p-phenylenediamine. |

By prolonging the duration of the exposure to the second target temperature, optional step G assists in crystallization of the MBB in high yields, thereby reducing yield loss by incomplete crystallization/precipitation. Suitably, the mixture is stirred while being maintained at the second target temperature. According to embodiments, the mixture is maintained in step G at the second target temperature for 10-60 minutes. Typically, maintaing the mixture in step G for 25-40 minutes at the second target temperature will be sufficient in order to achieve approximately complete crystallization. Conveniently, the mixture may be held at the second target temperature in step G for 30-35 minutes, for example about 30 minutes. Optional step G preferably is carried out in moisture-free conditions under an inert atmosphere.

In step H of the recrystallization method, the crystallized/precipitated MBB is collected by cold-filtering. The cold-filtering is carried out at a temperature at which the solubiliy of MBB in the solvent is sufficiently low in order to keep low potential yield loss due to the filtration step. According to embodiments, said cold-filtering in step H is done at the second target temperature, for example at a temperature of less than 8°C. According to embodiments, the cold-filtering in step H may be done at a temperature within the range of 0.0-5.0°C, in particular within the range of 0.0-4.0°C. According to specific embodiments, the cold-filtering in step H may be done at a temperature within the range of 0.0-3.0°C. For example, the cold-filtering in step H may be done at a temperature of about 3.0°C.

In order to further improve the purity of the recrystallized MBB obtained, filtration step H may comprise washing the collected MBB, in order to remove contaminants that may adhere to the surface of the MBB. According to embodiments, filtration step H optionally may comprise the indicated steps in numerical order:

| | |
|---|---|
| H1 | cold-filtering the filtrate, |
| H2 | cold-washing the filter residue with organic solvent comprising said aromatic solvent, and removing the washing liquid by filtering. |

The washing liquid used in optional step H2 exhibits a temperature below the MBB crystallization on-set temperature, and in particular may exhibit a temperature within the range of the second target temperature.

According to embodiments, the filter residue is washed in step H2 up to three times each with 0.5-1.0 parts-by-volume, in particular 0.7-0.9 parts-by-volume. For example, the filter residue may be washed in step H2 once or twice each with about 0.8 parts-by-volume of the organic solvent. According to embodiments, the filter residue may be washed in step H2 with a total of 1.0-2.0 parts-by-volume of the organic solvent. The washing liquid is removed by cold-filtering as described for step H.

Filtration step H, and optional steps H1-H2 preferably is/are carried out in moisture-free conditions under an inert atmosphere.

According to particular embodiments, the organic solvent used in step H2 as the washing liquid is a mixture of 65-100 vol% toluene and 35-0 vol% ethylacetate. For example, the washing liquid may be a mixture of 75-85 vol% toluene and 25-15 vol% ethylacetate. Mixtures of 80 vol% and 20 vol% ethylacetate have been found to be particularly suitable as a washing liquid.

The washing liquid may be combined with the filtrate (i.e. the removed aromatic solvent), at least provided the washing liquid does not interfere with recycling of the aromatic solvent, if possible at all. For example, if the aromatic solvent is toluene, which may be recycled, and the washing liquid is a mixture of toluene and ethylacetate, the washing liquid perferably is discarded, as it interferes with recycling of toluene.

The solid residue obtained in step H/H2 suitably may be dried. Drying suitably is effected under medium vacuum conditions at moderate temperatures. Temperatures of 50-70°C at a pressure of 2.0 mbar or less, in particular at 0.1-1.0 mbar have been found suitable for drying the recrystallized MBB. Drying is carried out routinely to a residual water content of 0.5 % w/w or less, in particular 0.1 or less, and a residual solvent content of 1000 ppm or less. Residual water is determined by Karl-Fisher-Titration, while residual solvent is determined by HPLC. Acceptable levels of residual water and residual solvent typically are achieved by drying for 5-8 hours at the indicated conditions.

As with the other steps of the method, drying preferably is carried out in moisture-free conditions under an inert atmosphere.

The method described above may be preceded, as an initial step, by determining whether or not a batch of MBB starting material comprises contaminants at levels that might be non-acceptable for cosmetic applications. Accordingly the method further may comprise the following step A as an initial step:

| | |
|---|---|
| A | determining at 525 nm the UV absorbance of an aqueous solution of the 2-methoxymethyl-p-phenylenediamine starting material. |

UV absorbance is determined in aqueous solution, and step A accordingly is not carried out in moisture-free conditions under an inert atmosphere. The measurement is carried out under standard conditions (ambient temperature and pressure) using a 10 mm pathway (cuvette). The concentration of the MBB is 1.0 PBW in a total volume of 100.0 PBV distilled water. In order to avoid excessive degradation of the MBB during the measurement, the MBB desirably is protected by the presence of an antioxidant in the solution. Suitable antioxidants include for example ascorbic acid and sulfites, such as sodium sulfite. Suitable amounts of antioxidant typically are within the range 25-50 parts-by-weight per 100 parts-by-weight MBB starting material. A solution to be used in the determination of the UV absorbance of MBB typically is prepared by dissolving the antioxidant first, and subsequently adding the required amount of MBB to the solution. A solution for determining the UV absorbance may be prepared, for example, by dissolving 0.30 g ascorbic acid in 80 ml distilled water in a 100 ml volumetric flask, adding 1.0 g MBB, completely dissolving the MBB, for example by sonication, and filling up with distilled water to 100 ml. The measurement then is carried out at 525 nm under standard conditions using a 10 mm pathway (cuvette).

The UV absorbance is determined at 525 nm. If the UV absorbance of the MBB tested at 525 nm is 0.0080 or above, the quality is non-acceptable for cosmetic applications, and the MBB is subjected to the recrystallization method. The UV absorbance determined in step A suitably may be used for estimating the amount of activated charcoal required in step B. If the UV absorbance obtained in step A exceeds 0.0500, using an amount of at least 8 parts-by-weight activated charcoal per 100 parts-by-weight MBB in step B might be considered.

The method described above further may comprise, as a final step, determining whether the recrystallized MBB obtained by the above described method exhibits cosmetic grade quality. Accordingly the method further may comprise the following step Z as an initial step:

| | |
|---|---|
| Z | determining at 525 nm the UV absorbance of an aqueous solution of the 2-methoxymethyl-p-phenylenediamine obtained. |

The UV absorbance is determined as described above for step A. If the UV absorbance of the MBB tested at 525 nm is less than 0.0080, the recrystallization method was successful, and the MBB obtained comprises cosmetic grade quality. When tested within 10 days after recovery of the MBB (steps H/H2), the MBB recrystallized according to the above method typically exhibits a UV absorbance at 525 nm of less than 0.0050.

The MBB obtained finally may be stored, packaged or subjected to subsequent processing. Storage or packaging preferably is done in moisture-free conditions, under an inert atmosphere, and protected from daylight.

### 2.2 High yield recrystallization method

According to the present invention, a method for converting colored 2-methoxymethyl-p-phenylenediamine starting material having an impurity degree non-acceptable for cosmetic applications to 2-methoxymethyl-p-phenylenediamine meeting the requirements for cosmetic applications comprises, the colored starting material being deteriorated by surface oxidation upon storage for several weeks or months:

| | |
|---|---|
| K | preparing a mixture of 100 parts-by-weight of the colored deteriorated 2-methoxymethyl-p-phenylenediamine starting material, activated charcoal, and a sufficient amount of non-aromatic solvent for dissolving the 2-methoxymethyl-p-phenylenediamine, |
| L | heating the mixture of step K to a third target temperature, |
| N | removing the activated charcoal by filtering, thereby obtaining a filtrate, |
| P | bringing the filtrate to a fourth target temperature, and under reduced pressure reducing the amount of non-aromatic solvent in the filtrate by distillation, |
| Q | adding aromatic solvent to the filtrate, |
| R | distilling the filtrate under reduced pressure at the fourth target temperature until obtaining a ratio of 700 or less parts-by-volume of organic solvent per 100 parts-by-weight of the 2-methoxymethyl-p-phenylenediamine starting material, |
| S | increasing the pressure to ambient pressure, and cooling the filtrate to a fifth target temperature, |
| U | filtering the filtrate at the fifth target temperature, thereby obtaining a residue of recrystallized 2-methoxymethyl-p-phenylenediamine. |

The non-aromatic solvent used in the recrystallization method is selected from methanol, ethanol, iso-propanol, n-propanol, ethyl formiate, methyl acetate, ethyl acetate, methyl propionate, ethyl propionate, CHCl₃, CCl₄, THF, 1,4-dioxane, or combinations thereof. Ethyl acetate is the presently preferred non-aromatic solvent.

The aromatic solvent used in the recrystallization method is selected from toluene, xylene, anisole, cresol, and combinations thereof. Toluene is the presently preferred aromatic solvent.

In order to protect MBB from oxidation, all of the above steps preferably are carried out in moisture-free conditions under an inert atmosphere. In other words, access of moisture and air oxygen to the reaction vessel and the end product obtained preferably is limited in order to reduce yield loss. Excluding oxygen and moisture/humidity suitably increases the yield of MBB. It is usually preferable to maintain moisture-free conditions and the presence of an inert atmosphere as much as possible.

A sufficient amount of the non-aromatic solvent for dissolving the colored MBB is used in step K of the recrystallization method. A sufficient amount of the non-aromatic solvent is within the range of 700-1200 parts-by-volume per 100 parts-by-weight MBB, typically within the range of 800-1100 parts-by-volume. More typically, the amount is within the range of 900-1000 parts-by-volume, in particular 930-980 parts-by-volume. For example, the amount of the non-aromatic solvent may be about 950 parts-by-volume per 100 parts-by-weight MBB, in particular if the non-aromatic solvent is ethyl acetate.

The mixture in step K is conveniently prepared by first dissolving the colored MBB starting material in the solvent, typically at ambient temperature (20-25°C) and ambient pressure, and subsequently adding the solution to the activated charcoal previously placed in the reaction vessel used for the recrystallization method, or vice versa. Alternatively, solvent, MBB and activated charcoal may be added essentially simultaneously to the reaction vessel used in the recrystallization method. Prior to adding MBB, the reaction vessel is preferably purged with inert gas.

The amount of activated charcoal used in step K typically is within the range of 3-20 parts-by-weight per 100 parts-by-weight MBB. The exact amount may be adjusted depending on the extent of coloration of the MBB starting material, or contamination level, respectively. For most contamination levels, 5-15 and for example 5-12, such as 5-10 parts-by-weight activated charcoal per 100 parts-by-weight MBB should be sufficient. Less than 3 parts-by-weight activated charcoal per 100 parts-by-weight MBB may not be sufficient for decreasing the levels of contaminants to the extent desired (or if it is sufficient, the time and effort for conducting the recrystallization method may not be justifed). Amounts of activated charcoal exceeding 20 parts-by-weight per 100 parts-by-weight MBB usually will not be required, except maybe for extremely high contamination levels. Since the activated charcoal adds to the expenses of the method, the amount actually added should be balanced with the actual level of contaminations.

The type of activated charcoal used in not specifically critical, and activated charcoals commercially available have been found suitable.

The mixture prepared in step K preferably is held in moisture-free conditions under an inert atmosphere. As such, the reaction vessel comprising the said mixture preferably is integrated into a closed system connected to a vacuum source and a supply of inert gas, which system is adapted to enable conducting preferably all steps of the recrystallization method in moisture-free conditions under an inert atmosphere.

The mixture of charcoal and MBB/solvent solution is heated in step L of the recrystallization method to a third target temperature. Suitably, the mixture is stirred while being heated to the third target temperature. It is understood that the heating rate is not particularly critical. Heating rates of 0.5-1.0°C per minute have been found suitable for heating the mixture of MBB, solvent and activated charcoal to a third target temperature. Heating rates below or above this range may be used, however, provided that the specific heating rate does not entail specific disadvantages such as for example an increased decomposition of the MBB.

The mixture of charcoal and MBB/solvent solution is heated in step L of the recrystallization method to a third target temperature. In the concentrations contemplated, the MBB is completely soluble in the non-aromatic solvent at the third target temperature and at ambient pressure. In other words, the third target temperature corresponds to the MBB crystallization on-set temperature or is a temperature higher than the MBB crystallization on-set temperature. Typically, the third target temperature is a temperature 10°C or more, suitably 15°C or more, and sometimes 20°C or more above the MBB crystallization on-set temperature". Suitably, the third target temperature is within the range of 50-100°C. According to embodiments, the third target temperature may be within the range of 70-90°C, for example within the range of 75-85°C. According to specific embodiments, the third target temperature may be within the range of 78-82°C, in particular if the non-aromatic solvent used is ethyl acetate. For example, the third target temperature may be about 80°C, in particular if the solvent is ethyl acetate. For non-aromatic solvents other than ethyl acetate, the third target temperature suitably is selected within a range above the MBB crystallization on-set temperature as descrived above, and below the boiling point of the solvent used. Step L preferably is carried out in moisture-free conditions under an inert atmosphere.

When the mixture has been brought to the third target temperature, the activated charcoal is removed by filtering. Prior to filtering, the method further may comprise optional step M:

| | |
|---|---|
| M | maintaining the mixture at the third target temperature for a time sufficient to at least partially adsorb dissolved impurities on the activated charcoal. |

By prolonging the duration of the exposure to the third target temperature in the presence of the activated charcoal, optional step M assists in absorbing more contaminants present in the mixture on the surface of the activated charcoal. Suitably, the mixture is stirred while being maintained at the third target temperature. According to embodiments, the mixture is maintained in step M at the third target temperature for 8-30 minutes. Typically, maintaing the mixture in step M for 8-15 minutes will be sufficient in order to achieve significant adsorption of contaminants present. Conveniently, the mixture may be held at the third target temperature in step M for 9-11 minutes, for example about 10 minutes. Optional step M preferably is carried out in moisture-free conditions under an inert atmosphere.

In step N of the recrystallization method, the activated charcoal is removed by filtering. The filtering is carried out at a temperature above the MBB crystallization on-set temperature, in order to reduce yield loss due to the filtration step. According to embodiments, the filtering in step N may be done at a temperature of at least 20°C, in particular at a temperature of at least 25°C. According to specific embodiments, the filtering in step E may be done at a temperature of at least 30°C, for example at a temperature of at least 40°C. According to embodiments, said filtering in step N is done at the third target temperature.

The activated charcoal filtered-off in step N may be discarded. The filtrate obtained after having removed the activated charcoal comprises the solution of MBB in the non-aromatic solvent, with a reduced level or amount of contaminants, as compared to the MBB starting material.

In order to further reduce yield loss due to the filtration step, filtration step N optionally may comprise washing the filtered-off activated charcoal. According to embodiments, filtration step N optionally may comprise the indicated steps in numerical order:

| | |
|---|---|
| N1 | removing the activated charcoal by filtering, |
| N2 | washing the filter residue with said non-aromatic solvent, and obtaining the washing liquid by filtering, |
| N3 | combining the washing liquid with the filtrate. |

The washing liquid used in optional step N2 exhibits a temperature above the MBB crystallization on-set temperature, and in particular a temperature of at least 20°C. For example, the washing liquid may exhibit a temperature within the range of the third target temperature.

According to embodiments, the filter residue is washed in step N2 up to three times each with 0.2-1.0 parts-by-volume, in particular 0.4-0.6 parts-by-volume. For example, the filter residue may be washed in step N2 twice or three times each with about 0.5 parts-by-volume of the non-aromatic solvent. According to embodiments, the filter residue may be washed in step N2 with a total of about 1.0 parts-by-volume of the non-aromatic solvent. The washing liquid is filtered off as described for step N, collected and combined with the filtrate.

Filtration step N, and optional steps N1-N3 preferably is/are carried out in moisture-free conditions under an inert atmosphere.

The recrystallization method further may comprise optional step O:

| | |
|---|---|
| O | adjusting the filtrate to a ratio of 1000-1500 parts-by-volume, in particular 1100-1300 parts-by-volume, such as 1150-1250 parts-by-volume non-aromatic solvent per 100 parts-by-weight of the 2-methoxymethyl-p-phenylenediamine starting material. |

Optional step O is carried out by adding non-aromatic solvent in the required amounts. Suitably, the filtrate is stirred while adding the non-aromatic solvent. Step O preferably is carried out in moisture-free conditions under an inert atmosphere. The volume of the non-aromatic solvent present, and its ratio in relation to the amount of MBB prior to step O depends on the volume of non-aromatic solvent initially added in step K, and the volume of washing liquid in step N2, if any. Optional step O brings the ratio MBB/solvent within specified ranges, and dilutes the MBB solution. Lower MBB concentrations tend to promote crystallization in the subsequent steps while avoiding premature or uncontrolled precipitation.

Step P of the recrystallization method brings the filtrate to a "fourth target temperature", which term is used herein to refer to denote conditions defining a temperature range at reduced pressure. "Fourth target temperature" conditions are as well maintained in the subsequent steps Q and R of the recrystallization method. The temperature is essentially held constant during steps P-Q, while the pressure is gradually further reduced.

The particular order of the temperature/pressure adjustment at the beginning of step P is not critical. The filtrate first may be brought to the fourth target temperature and the pressure be reduced subsequently, or vice versa. Alternatively, the pressure may be gradually reduced while bringing the filtrate to the fourth target temperature. Suitably, the filtrate is stirred during step P. Step P preferably is carried out in moisture-free conditions under an inert atmosphere.

At the beginning of step P, the fourth target temperature is a temperature at which the MBB in the actual concentration at the pressure involved is completely soluble in the non-aromatic solvent, i.e. a temperature above the MBB crystallization on-set temperature. While the pressure is reduced over the course of step P, the pressure end point in step P is a pressure at which the non-aromatic solvent boils at the fourth target temperature, so that the amount of the non-aromatic solvent is reduced by distillation. At the end of step P, the fourth target temperature is a temperature at which the MBB in the actual concentration at the pressure end point is close to the MBB crystallization on-set temperature, preferably within ±2.0°C of the MBB crystallization on-set temperature.

Suitably, the fourth target temperature may be within the range of 35-48°C at a pressure within the range of 80-300 mbar.

According to embodiments, the fourth target temperature at the beginning of step P may be within the range of 35-42°C at a pressure within the range of 120-280 mbar. For example, the fourth target temperature at the beginning of step P may be within the range of 36-40°C at a pressure within the range of 150-260 mbar.

Typical pressure end points for step P are in the order of 120-240 mbar, for example 150-225 mbar, at a temperature preferably within the range of 36-40°C. The pressure decline and pressure end point are suitably selected in order to reduce the amount of non-aromatic solvent in a controllable distillation. According to embodiments, the amount of non-aromatic solvent is reduced in step P to 400-600 parts-by-volume (per 100 parts-by-weight MBB). For example, the non-aromatic solvent may be reduced in step P to 420-500 parts-by-volume. According to a specific embodiment, the non-aromatic solvent may be reduced in step P to about 440 parts-by-volume. The filtrate suitably is stirred while removing the non-aromatic solvent. The non-aromatic solvent may be collected and subjected to recycling, if possible.

Temperature, pressure end point and ratio of non-aromatic solvent to MBB at the end of step P furthermore preferably are selected such that the MBB concentration is close to the concentration where MBB crystallization starts (MBB crystallization on-set concentration). In other words, at the end of step P the MBB dissolved in the non-aromatic solvent preferably just begins to precipitate or is close to the point where precipitation begins. If the conditions at the end of step P - prior to starting adding aromatic solvent in step Q - are selected such that MBB is close to its saturation limit, the concentration of seeding crystals in the solution is high. It has been found out that a high concentration of seeding crystals contributes to the formation of crystalline material when proceeding to the addition of aromatic solvent in step Q, so that the formation of amorphous material advantageously can be kept low. Avoiding excessive formation of amorphous material contributes to the quality of the final product obtained in the recrystallization method. According to embodiments, temperature, pressure and MBB concentration at the end of step P are selected such that MBB is within ±2.0°C, for example within ±1.5°C or within ±1.0°C of its crystallization on-set temperature.

In step Q of the method, aromatic solvent is added to the filtrate. The MBB has a lower solubility in the aromatic solvent, or in the mixture of aromatic and non-aromatic solvent, as compared to its solubility in the non-aromatic solvent alone. Typically, the end pressure and temperature from step P are not significantly changed when proceeding to step Q. More typically, end pressure and temperature from step P may be essentially maintained when proceeding to step Q.

Suitably, the filtrate is stirred while adding the aromatic solvent. Step Q preferably is carried out in moisture-free conditions under an inert atmosphere. According to embodiments, the aromatic solvent is added to the filtrate while continuing to distill the filtrate under reduced pressure at the fourth target temperature. According to embodiments, a gradual addition of aromatic solvent to the filtrate is started while continuing to distill the filtrate under reduced pressure at the fourth target temperature.

The amount of aromatic solvent added in step Q preferably is 500-700 parts-by-volume (per 100 parts-by-weight MBB). According to embodiments, the amount of aromatic solvent added in step Q may be 550-650 parts-by-volume. According to a specific embodiment, the amount of aromatic solvent added in step Q may be about 600 parts-by-volume.

The aromatic solvent added in step Q is selected from toluene, xylene, anisole, cresol, and combinations thereof. A presently preferred aromatic solvent to be added in step Q is toluene.

According to preferred embodiments, the aromatic solvent is added gradually in step Q. Conveniently, the aromatic solvent may be added dropwise to the filtrate. According to embodiments, gradual addition of the aromatic solvent to the filtrate is started while continuing to distill the filtrate under reduced pressure at the fourth target temperature. At the prevailing reduced pressure in step Q, the aromatic solvent may have a medium to high vapour pressure at the fourth target temperature. As a consequence thereof, the distillate removed in step Q may comprise a mixture of the non-aromatic solvent and the aromatic solvent. With a gradually increasing ratio of aromatic to non-aromatic solvent due to the gradual addition of aromatic solvent and depletion of the non-aromatic solvent by distillation, the ratio of aromatic to non-aromatic solvent in the distillate typically increases over the time.

For example, if ethyl acetate is the selected non-aromatic solvent and toluene is the selected aromatic solvent, the distillate in step Q will be an azeotrope of the two solvents. In such case, the distillate cannot be recycled and has to be discarded. If so, the distillate of step Q preferably is held separate from the distillate collected previously in step P.

According to embodiments, the addition rate of the aromatic solvent may be selected in step Q such that the ratio-by-volume of aromatic solvent added per time unit to organic solvent removed per time unit is within the range of 3.0-1.0. In other words, the volume increase by addition of the aromatic solvent is conter-balanced by a volume loss due to removal of organic solvent by distillation. At one end of the range the volume addition per time unit may essentially correspond to the volume loss per time unit. At the other end of the range, the volume added per time unit may correspond to about three times the volume distilled-off in the same time unit. According to embodiments, the addition rate of the aromatic solvent may be selected such that the ratio-by-volume of aromatic solvent added per time unit to organic solvent removed per time unit is within the range of 2.5-1.5. For example, the volume addition to volume loss ratio over the time may be about 2.0.

The fourth target temperature during step Q suitably may be within the range of 38-45°C at a pressure decreasing over the course of step Q from the pressure end point of step P by 20-50%, for example decreasing to 90-150 mbar. According to embodiments, the fourth target temperature during step Q may be within the range of 40-45°C, and the pressure end point in step Q may be within the range of 100-140 mbar.

When the addition of the aromatic solvent is completed, removing the organic solvent by distillation as done in step Q is continued until a specific ratio of organic solved to MBB is reached, denoted herein separately as step R. Typically, the pressure and temperature from step Q are not significantly changed when proceeding to step R. More typically, pressure and temperature from step Q may be essentially maintained when proceeding to step R. Suitably, the filtrate is stirred while continuing to remove the organic solvent. Step R preferably is carried out in moisture-free conditions under an inert atmosphere.

In step R, distilling the filtrate is continued until obtaining a ratio of 700 or less parts-by-volume of organic solvent per 100 parts-by-weight of the MBB starting material. According to embodiments, distilling the filtrate is continued until the ratio obtained is 500-550 parts-by-volume of organic solvent per 100 parts-by-weight of the MBB starting material. For example, distilling the filtrate may be continued to a ratio of 500-520 parts-by-volume of organic solvent per 100 parts-by-weight of the MBB starting material.

With continuing distillation, the organic solvent gradually becomes enriched in the solvent component having the lower vapour pressure while becoming depleted in the component having the higher vapour pressure. As a consequence, the distillation speed (solvent volume removed per time unit by distillation) decreases over the time when holding constant temperature and pressure. According to embodiments, the temperature is maintained within the fourth target temperature during steps P-R of the recrystallization method. In particular, the temperature may be maintained essentially constant during steps P-R, or may be allowed to increase moderately within the range of the fourth target temperature. Vice versa, when holding the temperature during steps P-R within the fourth target temperature, the distillation speed may be controlled by adjusting the pressure. In particular, the pressure may be further reduced from the values indicated for step P during the course of the further distillation in order to maintain the distillation speed within desired limits. For example, the pressure may be reduced during steps R and Q to keep the distillation speed essentially constant.

The fourth target temperature during step R suitably may be within the range of 40-48°C at a pressure optionally decreasing over the course of step R from the pressure end point of step Q by 0-20%. According to embodiments, the fourth target temperature during step R may be within the range of 41-46°C, and the pressure end point in step R may be within the range of 80-140 mbar.

When a ratio of 700 or less parts-by-volume of organic solvent per 100 parts-by-weight of the MBB starting material is obtained at the end of step R, the concentration of the non-aromatic solvent is reduced to an extent allowing for recrystallization of MBB with low yield loss. The ratio of organic solvent to MBB starting material conveniently can be determined in view of the known volumes of starting materials in step K, solvent volume added in steps N3, O and Q, and solvent volume distilled-off in steps P-R (and the volume remaining in step R).

When the ratio of 700 or less parts-by-volume of organic solvent per 100 parts-by-weight of the MBB starting material is obtained, the vacuum is relaxed to ambient pressure and the contents of the reaction vessel (irrespective of any intervening steps still denoted herein as "filtrate") are cooled to a fifth target temperature in step S of the recrystallization method. The particular order of the temperature/pressure adjustment in step S is not critical. The pressure may be increased first, and the filtrate may be brought to the fifth target temperature subsequently, or vice versa. Alternatively, the pressure may be gradually increased while bringing the filtrate to the fifth target temperature. Suitably, the filtrate is stirred during step S. Step S preferably is carried out in moisture-free conditions under an inert atmosphere.

Relaxing the vacuum from reduced pressure to ambient pressure and cooling to the fifth target temperature both promotes the precipitation or crystallization of MBB. Changing the two parameters preferably is done sufficiently slow in order to allow for the formation of crystals, and essentially avoiding the formation of amorphous material. The more important parameter in this tyoically is the pressure increase. According to embodiments, the pressure is increased in step S at a rate of 0.1-10 mbar per second. Cooling rates of 0.5-2.0°C per minute have been found suitable for cooling the filtrate to the fifth target temperature in step S. According to embodiments, the cooling rate to the fifth target temperature in step S is 0.5-1.0°C per minute. Cooling the filtrate may comprise active cooling, or allowing the filtrate to cool without active cooling.

As the filtrate is cooled to the fifth target temperature, the initial clear filtrate turns into a suspension due to the formations of MBB crystals. Any crystals sticking to the wall of the reaction vessel may be removed mechanically by scraping-off using appropriate devices of the system.

The fifth target temperature is a temperature at which the solubiliy of MBB in the solvent is sufficiently low in order to keep low potential yield loss due to the subsequent filtration step. Suitably, the fifth target temperature is less than 6.0°C. According to embodiments, the fifth target temperature may be within the range of 0.0-4.0°C, in particular within the range of 0.0-3.0°C. According to specific embodiments, the fifth target temperature may be within the range of 0.0-2.5°C.

When the filtrate has been brought to the fifth target temperature, the MBB is collected as a solid residue by filtering, thereby removing the solvent. Prior to filtering, the method further may comprise optional step T:

| | |
|---|---|
| T | maintaining the filtrate at the fifth target temperature to allow for precipitation of 2-methoxymethyl-p-phenylenediamine. |

By prolonging the duration of the exposure to the fifth target temperature, optional step T assists in crystallization of the MBB in high yields, thereby reducing yield loss by incomplete crystallization/precipitation. Suitably, the mixture is stirred while being maintained at the fifth target temperature. According to embodiments, the mixture is maintained in step T at the fifth target temperature for 10-60 minutes. Typically, maintaing the mixture in step T for 25-40 minutes at the fifth target temperature will be sufficient in order to achieve approximately complete crystallization. Conveniently, the mixture may be held at the fifth target temperature in step T for 30-35 minutes, for example about 30 minutes. Optional step T preferably is carried out in moisture-free conditions under an inert atmosphere.

In step U of the recrystallization method, the crystallized/precipitated MBB is collected by cold-filtering. The cold-filtering is carried out at a temperature at which the solubiliy of MBB in the solvent is sufficiently low in order to keep low potential yield loss due to the filtration step. According to embodiments, said cold-filtering in step U is done at the fifth target temperature, for example at a temperature of less than 6.0°C. According to embodiments, the cold-filtering in step U may be done at a temperature within the range of 0.0-4.0°C, in particular within the range of 0.0-3.0°C. According to specific embodiments, the cold-filtering in step U may be done at a temperature within the range of 0.0-2.5°C.

In order to further improve the purity of the recrystallized MBB obtained, filtration step U may comprise washing the collected MBB, in order to remove contaminants that may adhere to the surface of the MBB. According to embodiments, filtration step U optionally may comprise the indicated steps in numerical order:

| | |
|---|---|
| U1 | cold-filtering the filtrate, |
| U2 | cold-washing the filter residue with organic solvent comprising said aromatic solvent, and removing the washing liquid by filtering. |

The washing liquid used in optional step U2 exhibits a temperature below the MBB crystallization on-set temperature, and in particular may exhibit a temperature within the range of the fifth target temperature.

According to embodiments, the filter residue is washed in step U2 up to three times each with 0.5-1.0 parts-by-volume, in particular 0.6-0.8 parts-by-volume. For example, the filter residue may be washed in step U2 once or twice each with about 0.7 parts-by-volume of the organic solvent. According to embodiments, the filter residue may be washed in step U2 with a total of 1.0-2.0 parts-by-volume of the organic solvent. The washing liquid is removed by cold-filtering as described for step U.

Filtration step U, and optional steps U1-U2 preferably is/are carried out in moisture-free conditions under an inert atmosphere.

According to particular embodiments, the organic solvent used in step U2 as the washing liquid is a mixture of 65-100 vol% toluene and 35-0 vol% ethylacetate. For example, the washing liquid may be a mixture of 75-85 vol% toluene and 25-15 vol% ethylacetate. Mixtures of 80 vol% and 20 vol% ethylacetate have been found to be particularly suitable as a washing liquid.

The solid residue obtained in step U/U2 suitably may be dried. Drying suitably is effected under medium vacuum conditions at moderate temperatures. Temperatures of 50-70°C at a pressure of 2.0 mbar or less, in particular at 0.1-1.0 mbar have been found suitable for drying the recrystallized MBB. Drying is carried out routinely to a residual water content of 0.5 % w/w or less, in particular 0.1 or less, and a residual solvent content of 1000 ppm or less. Residual water is determined by Karl-Fisher-Titration, while residual solvent is determined by HPLC. Acceptable levels of residual water and residual solvent typically are achieved by drying for 5-8 hours at the indicated conditions.

As with the other steps of the method, drying preferably is carried out in moisture-free conditions under an inert atmosphere.

The method described above may be preceded, as an initial step, by determining whether or not a batch of MBB starting material comprises contaminants at levels that might be non-acceptable for cosmetic applications. Accordingly the method further may comprise the following step A as an initial step:

| | |
|---|---|
| A | determining at 525 nm the UV absorbance of an aqueous solution of the 2-methoxymethyl-p-phenylenediamine starting material. |

UV absorbance is determined in aqueous solution, and step A accordingly is not carried out in moisture-free conditions under an inert atmosphere. The measurement is carried out under standard conditions (ambient temperature and pressure) using a 10 mm pathway (cuvette). The concentration of the MBB is 1.0 PBW in a total volume of 100.0 PBV distilled water. In order to avoid excessive degradation of the MBB during the measurement, the MBB desirably is protected by the presence of an antioxidant in the solution. Suitable antioxidants include for example ascorbic acid and sulfites, such as sodium sulfite. Suitable amounts of antioxidant typically are within the range 25-50 parts-by-weight per 100 parts-by-weight MBB starting material. A solution to be used in the determination of the UV absorbance of MBB typically is prepared by dissolving the antioxidant first, and subsequently adding the required amount of MBB to the solution. A solution for determining the UV absorbance may be prepared, for example, by dissolving 0.30 g ascorbic acid in 80 ml distilled water in a 100 ml volumetric flask, adding 1.0 g MBB, completely dissolving the MBB, for example by sonication, and filling up with distilled water to 100 ml. The measurement then is carried out at 525 nm under standard conditions using a 10 mm pathway (cuvette).

The UV absorbance is determined at 525 nm. If the UV absorbance of the MBB tested at 525 nm is 0.0080 or above, the quality is non-acceptable for cosmetic applications, and the MBB is subjected to the recrystallization method. The UV absorbance determined in step A suitably may be used for estimating the amount of activated charcoal required in step K. If the UV absorbance obtained in step A exceeds 0.0500, using an amount of at least 8 parts-by-weight activated charcoal per 100 parts-by-weight MBB in step K might be considered.

The method described above further may comprise, as a final step, determining whether the recrystallized MBB obtained by the above described method exhibits cosmetic grade quality. Accordingly the method further may comprise the following step Z as an initial step:

| | |
|---|---|
| Z | determining at 525 nm the UV absorbance of an aqueous solution of the 2-methoxymethyl-p-phenylenediamine obtained. |

The UV absorbance is determined as described above for step A. If the UV absorbance of the MBB tested at 525 nm is less than 0.0080, the recrystallization method was successful, and the MBB obtained comprises cosmetic grade quality. When tested within 10 days after recovery of the MBB (steps U/U2), the MBB recrystallized according to the above method typically exhibits a UV absorbance at 525 nm of less than 0.0035.

The MBB obtained finally may be stored, packaged or subjected to subsequent processing. Storage or packaging preferably is done in moisture-free conditions, under an inert atmosphere, and protected from daylight.

### 3 2-Methoxymethyl-p-phenylenediamine in cosmetic grade quality

According to an aspect, the present invention relates to MBB in cosmetic quality, which MBB has been recrystallized by a method according to the present invention. The recrystallized MBB exhibits a UV absorbance at 525 nm of less than 0.0080. The recrystallized MBB obtained by a method according to the present invention may exhibit a UV absorbance at 525 nm of less than 0.0060, for example less than 0.0045, determined without 10 days after recovery of the recrystallized MBB in step H/H2 or U/U2.

According to another aspect, the present invention relates to MBB having cosmetic grade quality, as assessed by determining at 525 nm the UV absorbance of an aqueous solution of the MBB.

UV absorbance is determined in aqueous solution. The measurement is carried out under standard conditions (ambient temperature and pressure) using a 10 mm pathway (cuvette). The concentration of the MBB is 1.0 PBW in a total volume of 100.0 PBV distilled water. In order to avoid excessive degradation of the MBB during the measurement, the MBB desirably is protected by the presence of an antioxidant in the solution. Suitable antioxidants include for example ascorbic acid and sulfites, such as sodium sulfite. Suitable amounts of antioxidant typically are within the range 25-50 parts-by-weight per 100 parts-by-weight MBB starting material. A solution to be used in the determination of the UV absorbance of MBB typically is prepared by dissolving the antioxidant first, and subsequently adding the required amount of MBB to the solution. A solution for determining the UV absorbance may be prepared, for example, by dissolving 0.30 g ascorbic acid in 80 ml distilled water in a 100 ml volumetric flask, adding 1.0 g MBB, completely dissolving the MBB, for example by sonication, and filling up with distilled water to 100 ml. The measurement then is carried out at 525 nm under standard conditions using a 10 mm pathway (cuvette). A UV absorbance at 525 nm of less than 0.0080 indicates cosmetic grade quality of the MBB. According to embodiments, the MBB having cosmetic grade quality may exhibit a UV absorbance at 525 nm of less than 0.0060, for example less than 0.0050.

According to yet another aspect, the present invention relates to to MBB having a shelf-life of at least 9 months, preferably of at least 12 months under standard storage conditions. Standard storage conditions encompass temperatures of at most 25°C and moisture/humidity of 45% or less. The shelf-life of MBB may be defined in terms of its UV absorbance at 525 nm. For example, the MBB having a shelf-life of at least 9 months under standard storage conditions may exhibit a UV absorbance at 525 nm of less than 0.0048, for example less than 0.0045. For example, the MBB having a shelf-life of at least 12 months under standard storage conditions may exhibit a UV absorbance at 525 nm of less than 0.0035, for example less than 0.0032.

### EXAMPLES

The methods described below represent dedicated recrystallization procedures leaving sufficient freedom grades for the operator to decide, depending on the magnitude of contaminants present and depending on the amount of MBB to be recrystallized (e.g. if only one batch is affected or a large production run needs to be recrystallized), which method to chose as both methods differentiate regarding the complexity of using a mixture of solvents versus one single solvent coming along with specific advantages and certain downsides to be considered.

### Method A

The method described below is prefectly suitable to handle the recrystallization of MBB in an especially beneficial and economical way for large scale operation. The method is straightforward, uses only one type of solvent with low process complexity, and allows for recycling of the solvent.

Advantages here is the recovery of the single solvent (here: toluene) by distillation within a sustainable circulation in a continuous way. The method is easy to setup, fast and provides sustainable good results while the turnaround recrystallization can be comfortably carried out in one day.

As a matter of fact, while using only toluene as single solvent, the mixture is lacking some required polarity moves to keep the crystals during the crystallization process in homogenous suspension. Moderate and unavoidable crystal formation at the inner wall of the reaction vessel was observed in a range of about 10 - 15 % w/w. However, if multiple lots of MBB showing comparable impurity levels shall be recrystallized, this observation does not significantly impact the overall success of the procedure, especially in respect of economics, as the vessel can be repeatidly used in circles without cleaning in-between, and the precipitated crystal residue inside the vessel from previous batches can be routed directly to the next batch operation without any loss. Another observation which should be considered is the fact that the isolated final product of this method may appear as moderate brittle chunks after the drying process. Hence, the isolated product may require an additional grinding treatment to obtain a fine powder according to the performance specifications to be used in hair color formulations.

### Experimental procedure

All operations are carried out in moisture free conditions and are operated under a nitrogen atmosphere to avoid any access of oxygen.

1 part-by-weight MBB and 5-10 % w/w activated charcoal are suspended in 7 parts-by-volume toluene and heated to 80°C. After stirring for further 10 minutes at this temperature, the hot suspension is filtered. The filter residue is then washed twice with 0.5 parts-by-volume toluene having a temperature of 60°C or above. The mother liquid and the washing liquid are then combined and the united fraction is cooled to 0-5°C. The crystallization of MBB already starts at ca. 55°C and forms a reddish pale suspension. After stirring the cool suspension at 0-5°C for further 30 minutes, the reaction mixture is filtered-off. The obtained pale white/beige filter residue is washed with additional 0.8 parts-by-volume toluene. The residue is then dried at 60°C for 6 hours under vacuum conditions (0.2 mbar) and nitrogen atmosphere. The yield is up to 90-92% by weight. The UV absorbance at 525 nm is 0.0045.

Net, the recovery rate of MBB, based on the weight of the MBB starting material, can be confirmed to be reliably at approximately 90% by weight.

### Method B

The method described below is prefectly suitable to handle the recrystallization of MBB for few or even single batches of MBB contaminated. The method delivers competitive yields up to 93% by weight without any material loss caused by adsorption on the inner wall of the reaction vessel. As this method uses a solvent mixture (shown here: ethyl acetate and toluene) to effect precipitation of MBB, the ideal polarity required to avoid premature precipitation of MBB in the suspension can be achieved on the spot to keep the crystals at any time in homogenous suspension.

While no precipitation is formed during the method prior to cooling and starting the controlled precipitation, the final precipitate appears as fine and homogenous crystalline powder which avoids that the product can form brittle chunks, hence, no further milling or grinding is required to obtain a ready-for-use material. However, the finally obtained solvent mixture comprising toluene and ethyl acetate cannot be recycled, as the two solvents form at any temperature and pressure an azeotropic media which does not allow to separate the fractions to effectively recycle the two solvents. Anyway, method B represents a viable alternative to method A, as it allows to quickly recrystallize single batches of MBB if time is a critical economical factor.

The following section describes predominantly the crystallisation of MBB according to a lab scale procedure and focuses also on the details of the crystallization steps as this is the most important experimental part. Any upscaling was also carried out and it can be confirmed that the procedure is scalable with considerable ease for those skilled in the art without any adoptions, hence, the method described herein is valid for any scales.

### Experimental procedure

50 g (1 part-by-weight) (0.3285 mol) MBB comprising contaminants and 5-10 % w/w activated charcoal are suspended in 475 ml ethyl acetate (9.5 parts-by-volume). The MBB starts to dissolve while stirring the suspension and a temperature dropdown to approximately 5°C is observed. While heating to ambient temperature, the remaining MBB dissolves completely and a clear solution is formed at approximately 18°C. The solution subsequently is heated to 80°C. After stirring for further 10 minutes at this temperature, the hot suspension is filtered. The filter residue (activated charcoal) is washed twice with small portions of ethyl acetate. The filtrate and the washing liquid are combined.

After addition of 120 ml ethyl acetate (2.4 parts-by-volume), reduced pressure of approximately 240 mbar is applied to the reaction vessel and the reaction mixture is heated to reflux while the external heating was set to 58°C, resulting in a temperature inside the vessel of approximately 38°C. The solvent is removed by distillation until the volume of solvent removed corresponds to approximately 375 ml (7.5 parts-by-volume). During the course of that processing, the reaction pressure is gradually reduced while the reaction temperature is maintained in a controlled manner within a range of 35-39°C. Important to mention that the reaction temperature is the most important parameter to control the distillation process while controlling the vaccum and the distillation speed.

### Distillation protocol:

| reaction temperature (± 0.25°C) | pressure (± 2.5 mbar) | volume of ethyl acetate removed (± 1 ml) |
|---|---|---|
| 38 °C | 240 mbar | start of distillation distillate turbid due to water content |
| 39 °C | 240 mbar | 50 ml |
| 39 - 40 °C | 240 mbar | 50 ml |
| 38 °C | 230 mbar | 50 ml |
| 37 °C | 210 mbar | 50 ml |
| 38 °C | 210 mbar | 50 ml |
| 38 - 39 °C | 210 mbar | 50 ml |
| 36 - 37 °C | 190 mbar | 50 ml |
| 37 °C | 185 mbar | 25 ml |
| **total** | | **375 ml*** |

| | | |
|---|---|---|
| * accurate volume: 380 ml ≙ 7.6 parts-by-volume | | |

In the course of the distillation progress according to the above protocol the operation reaches a reaction temperature of 37°C and a vacuum of approximately 185 mbar. Approximately 375 ml of ethyl acetate distillate could be already collected while gradually a slow crystallization of MBB is observed. At this point in time, the addition of 300 ml toluene (6 parts-by-volume) is allowed to start. The addition speed of toluene is controlled carefully to reach an addition rate which is approximately twice the removal rate of the solvent. The distillation is continued while the reaction temperature is allowed to increase slowly up to 45°C which marks the end-point of the distilallation while the the majority of the MBB is precipitated.

### Distillation protocol, continued:

| reaction temperature (± 0.25°C) | pressure (± 2.5 mbar) | toluene added | volume of organic solvent removed (mixture of ethyl acetate / toluene) (± 1 ml) |
|---|---|---|---|
| 41 °C | 185 mbar | | 45 ml |
| 40 °C | 170 mbar | +150 ml | 45 ml |
| 42 °C | 140 mbar | +70 ml | 35 ml |
| 41 °C | 120 mbar | +80 ml | |
| 42 °C | 120 mbar | | 50 ml |
| 42 °C | 108 mbar | | 45 ml |
| 45 °C | 120 mbar | | no further distillate to observe |
| **total** | | **300 ml** | **265 ml*** |

| | | | |
|---|---|---|---|
| * accurate volume: 267 ml ≙ 5.34 parts-by-volume | | | |

| | |
|---|---|
| volume of distillate in total: | 380 ml + 267 ml = 647 ml ≙ 12.94 parts-by-volume |
| All measured values are real values, checked against calibrated temperature and pressure measurement devices | |

The distillation was finished when in total approximately 647 ml of distillate was collected which corresponds to 12.94 parts-by-volume. At this point in time, the temperature increased to a maximum of 45°C at an applied vacuum in the range of 110-120 mbar.

The reaction vessel is flooded with nitrogen while removing the vacuum and the suspension is finally cooled to 0-3°C. At 0-3°C, the suspension is further stirred for a period of approximately 30 minutes and subsequently filtered-off. The obtained residue is washed three times with toluene (0.7 parts-by-volume for each washing operation). The first washing liquid may optionally include the rinsing of the vessel to improve the efficiency also in terms of ecological manners to minimize organic waste. The obtained powder is dried at 60°C under vacuum.
Yield: 46,88g (corresponding to 93,76% in theory)
UV absorbance at 525 nm: 0.0030

## Claims

1. A method for assessing the quality of 2-methoxymethyl-p-phenylenediamine, comprising:
i determining at 525 nm the UV absorbance of an aqueous solution of 2-methoxymethyl-p-phenylenediamine,
ii grading the quality of the 2-methoxymethyl-p-phenylenediamine on basis of the UV absorbance obtained,
optionally wherein the aqueous solution of step (i) comprises an antioxidant.

2. A method for converting colored 2-methoxymethyl-p-phenylenediamine starting material having an impurity degree non-acceptable for cosmetic applications to 2-methoxymethyl-p-phenylenediamine meeting the requirements for cosmetic applications, the colored starting material being deteriorated by surface oxidation upon storage for several weeks or months, the method comprising the indicated steps in an alphabetical order:
| | |
|---|---|
| B | preparing a mixture of 100 parts-by-weight of the colored deteriorated 2-methoxymethyl-p-phenylenediamine starting material, activated charcoal, and a sufficient amount of aromatic solvent for dissolving the 2-methoxymethyl-p-phenylenediamine, |
| C | heating the mixture of step B to a first target temperature, |
| E | removing the activated charcoal by hot-filtering, thereby obtaining a filtrate, |
| F | cooling the filtrate to a second target temperature, |
| H | cold-filtering the filtrate, thereby obtaining a residue of recrystallized 2-methoxymethyl-p-phenylenediamine, |
wherein the aromatic solvent is selected from toluene, xylene, anisole, cresol, and combinations thereof.

3. The method of claim 2, wherein in step B the amount of activated charcoal is 3-20 parts-by-weight per 100 parts-by-weight 2-methoxymethyl-p-phenylenediamine starting material, in particular wherein the amount of activated charcoal in step B is 5-10 parts-by-weight per 100 parts-by-weight 2-methoxymethyl-p-phenylenediamine starting material, optionally wherein in step B the amount of aromatic solvent is within the range of 500-900 parts-by-volume, or 600-800 parts-by-volume, in particular 650-750 parts-by-volume, such as 680-720 parts-by-volume.

4. The method of claim 2 or 3, wherein the first target temperature is within the range of 60-100°C, in particular within the range of 70-90°C, for example within the range of 75-85°C, such as within the range of 78-82°C, and/or wherein the second target temperature is less than 8°C, for example within the range of 0.0-5.0°C, in particular 0.0-4.0°C, such as 0.0-3.0°C.

5. A method for converting colored 2-methoxymethyl-p-phenylenediamine starting material having an impurity degree non-acceptable for cosmetic applications to 2-methoxymethyl-p-phenylenediamine meeting the requirements for cosmetic applications, the colored starting material being deteriorated by surface oxidation upon storage for several weeks or months, the method comprising the indicated steps in an alphabetical order:
| | |
|---|---|
| K | preparing a mixture of 100 parts-by-weight of the colored deteriorated 2-methoxymethyl-p-phenylenediamine starting material, activated charcoal, and a sufficient amount of non-aromatic solvent for dissolving the 2-methoxymethyl-p-phenylenediamine, |
| L | heating the mixture of step K to a third target temperature, |
| N | removing the activated charcoal by filtering, thereby obtaining a filtrate, |
| P | bringing the filtrate to a fourth target temperature, and under reduced pressure reducing the amount of non-aromatic solvent in the filtrate by distillation, |
| Q | adding aromatic solvent to the filtrate, |
| R | distilling the filtrate under reduced pressure at the fourth target temperature until obtaining a ratio of 700 or less parts-by-volume of organic solvent per 100 parts-by-weight of the 2-methoxymethyl-p-phenylenediamine starting material, |
| S | increasing the pressure to ambient pressure, and cooling the filtrate to a fifth target temperature, |
| U | filtering the filtrate at the fifth target temperature, thereby obtaining a residue of recrystallized 2-methoxymethyl-p-phenylenediamine, |
wherein the non-aromatic solvent is selected from methanol, ethanol, iso-propanol, n-propanol, ethyl formiate, methyl acetate, ethyl acetate, methyl propionate, ethyl propionate, CHCl₃, CCl₄, THF, 1,4-dioxane, or combinations thereof,
wherein the aromatic solvent is selected from toluene, xylene, anisole, cresol, and combinations thereof.

6. The method of claim 5, wherein in step K the amount of activated charcoal is 3-20 parts-by-weight per 100 parts-by-weight 2-methoxymethyl-p-phenylenediamine starting material, in particular wherein the amount of activated charcoal in step K is 5-10 parts-by-weight per 100 parts-by-weight 2-methoxymethyl-p-phenylenediamine starting material.

7. The method of claim 5 or 6, wherein in step K the amount of non-aromatic solvent is within the range of 700-1200 parts-by-volume, or 800-1100 parts-by-volume, in particular 900-1000 parts-by-volume, such as 930-980 parts-by-volume.

8. The method of any of claims 5-7, wherein the third target temperature is within the range of 50-100°C, in particular within the range of 70-90°C, for example within the range of 75-85°C, such as within the range of 78-82°C, and/or wherein the fourth target temperature is within the range of 35-48°C at a pressure within the range of 120-280 mbar, and/or wherein the fifth target temperature is a temperature below 6.0°C, in particular within the range of 0.0-4.0°C, for example 0.0-3.0°C, such as 0.0-2.5°C.

9. The method of any of claims 5-8, further comprising step O:
| | |
|---|---|
| O | adjusting the filtrate to a ratio of 1000-1500 parts-by-volume, in particular 1100-1300 parts-by-volume, such as 1150-1250 parts-by-volume non-aromatic solvent per 100 parts-by-weight of the 2-methoxymethyl-p-phenylenediamine starting material. |

10. The method of any of claims 5-9, wherein in step Q the ratio-by-volume of aromatic solvent added per time unit to organic solvent removed per time unit is within the range of 3.0-1.0, in particular within the range of 2.5-1.5, such as about 2.0.

11. The method of any of claims 5-10, wherein the ratio obtained in step R is 500-600 parts-by-volume of organic solvent per 100 parts-by-weight of the 2-methoxymethyl-p-phenylenediamine starting material, in particular wherein the ratio is 500-550 parts-by-volume of organic solvent per 100 parts-by-weight of the 2-methoxymethyl-p-phenylenediamine starting material, for example wherein the ratio is 500-520 parts-by-volume of organic solvent per 100 parts-by-weight of the 2-methoxymethyl-p-phenylenediamine starting material.

12. The method of any of claims 5-11, wherein in steps P-R the temperature is maintained within the fourth target temperature, and the distillation speed is controlled by adjusting the pressure.

13. The method of any of claims 5-12, wherein the non-aromatic solvent is ethyl acetate, and/or wherein the aromatic solvent is toluene.

14. The method of any of claims 2-13, wherein each of steps B-H, and K-U is carried out in moisture-free conditions under an inert atmosphere.

15. The method of any of claims 2-14, further comprising step A and/or step Z:
| | |
|---|---|
| A | determining at 525 nm the UV absorbance of an aqueous solution of the 2- |
| | methoxymethyl-p-phenylenediamine starting material, |
| Z | determining at 525 nm the UV absorbance of an aqueous solution of the 2-methoxymethyl-p-phenylenediamine obtained. |

## Patentansprüche

1. Verfahren zum Bewerten der Qualität von 2-Methoxymethyl-p-phenylendiamin, umfassend:
i Bestimmen der UV-Absorption einer wässrigen Lösung von 2-Methoxymethyl-p-phenylendiamin bei 525 nm,
ii Einstufen der Qualität des 2-Methoxymethyl-p-phenylendiamins auf Basis der erhaltenen UV-Absorption,
optional wobei die wässrige Lösung aus Schritt (i) ein Antioxidans enthält.

2. Verfahren zum Überführen von verfärbtem 2-Methoxymethyl-p-phenylendiamin-Ausgangsmaterial mit einem für kosmetische Anwendungen nicht akzeptablen Verunreinigungsgrad in 2-Methoxymethyl-p-phenylendiamin, welches die Anforderungen für kosmetische Anwendungen erfüllt, wobei das verfärbte Ausgangsmaterial durch Oberflächenoxidation nach Lagerung über mehrere Wochen oder Monate hinweg verschlechtert ist, wobei das Verfahren die angegebenen Schritte in alphabetischer Reihenfolge umfasst:
| | |
|---|---|
| B | Herstellen eines Gemisches von 100 Gewichtsteilen des verfärbten, verschlechterten 2-Methoxymethyl-p-phenylendiamin-Ausgangsmaterials, Aktivkohle und einer ausreichenden Menge an aromatischem Lösungsmittel zum Auflösen des 2-Methoxymethyl-p-phenylendiamins, |
| C | Erwärmen des Gemisches aus Schritt B auf eine erste Zieltemperatur, |
| E | Entfernen der Aktivkohle durch Heißfiltration, wodurch ein Filtrat erhalten wird, |
| F | Abkühlen des Filtrats auf eine zweite Zieltemperatur, |
| H | Kaltfiltrieren des Filtrats, wodurch ein Rückstand von rekristallisiertem 2-Methoxymethyl-p-phenylendiamin erhalten wird, |
wobei das aromatische Lösungsmittel ausgewählt wird aus Toluol, Xylol, Anisol, Kresol, und Kombinationen davon.

3. Verfahren nach Anspruch 2, wobei in Schritt B die Menge an Aktivkohle 3-20 Gewichtsteile pro 100 Gewichtsteile 2-Methoxymethyl-p-phenylendiamin-Ausgangsmaterial beträgt, insbesondere wobei die Menge an Aktivkohle in Schritt B 5-10 Gewichtsteile pro 100 Gewichtsteile 2-Methoxymethyl-p-phenylendiamin-Ausgangsmaterial beträgt, optional wobei in Schritt B die Menge an aromatischem Lösungsmittel innerhalb des Bereichs von 500-900 Volumenteilen oder 600-800 Volumenteilen liegt, insbesondere 650-750 Volumenteile, wie etwa 680-720 Volumenteile.

4. Verfahren nach Anspruch 2 oder 3, wobei die erste Zieltemperatur innerhalb des Bereichs von 60-100°C liegt, insbesondere innerhalb des Bereichs von 70-90°C, beispielsweise innerhalb des Bereichs von 75-85°C, wie etwa innerhalb des Bereichs von 78-82°C, und/oder wobei die zweite Zieltemperatur weniger als 8°C beträgt, beispielsweise innerhalb des Bereichs von 0,0-5,0°C, insbesondere 0,0-4,0°C, wie etwa 0,0-3,0°C.

5. Verfahren zum Überführen von verfärbtem 2-Methoxymethyl-p-phenylendiamin-Ausgangsmaterial mit einem für kosmetische Anwendungen nicht akzeptablen Verunreinigungsgrad in 2-Methoxymethyl-p-phenylendiamin, welches die Anforderungen für kosmetische Anwendungen erfüllt, wobei das verfärbte Ausgangsmaterial durch Oberflächenoxidation nach Lagerung über mehrere Wochen oder Monate hinweg verschlechtert ist, wobei das Verfahren die angegebenen Schritte in alphabetischer Reihenfolge umfasst:
| | |
|---|---|
| K | Herstellen eines Gemisches von 100 Gewichtsteilen des verfärbten, verschlechterten 2-Methoxymethyl-p-phenylendiamin-Ausgangsmaterials, Aktivkohle und einer ausreichenden Menge an nicht-aromatischem Lösungsmittel zum Auflösen des 2-Methoxymethyl-p-phenylendiamins, |
| L | Erwärmen des Gemisches aus Schritt K auf eine dritte Zieltemperatur, |
| N | Entfernen der Aktivkohle durch Filtration, wodurch ein Filtrat erhalten wird, |
| P | Bringen des Filtrats auf eine vierte Zieltemperatur, und, unter vermindertem Druck, Verringern der Menge an nicht-aromatischem Lösungsmittel im Filtrat durch Destillation, |
| Q | Zugeben von aromatischem Lösungsmittel zum Filtrat, |
| R | Destillieren des Filtrats unter vermindertem Druck bei der vierten Zieltemperatur, bis ein Verhältnis von 700 oder weniger Volumenteilen an organischem Lösungsmittel pro 100 Gewichtsteilen des 2-Methoxymethyl-p-phenylendiamin-Ausgangsmaterials erreicht ist, |
| S | Erhöhen des Drucks auf Umgebungsdruck und Abkühlen des Filtrats auf eine fünfte Zieltemperatur, |
| U | Filtrieren des Filtrats bei der fünften Zieltemperatur, wodurch ein Rückstand von rekristallisiertem 2-Methoxymethyl-p-phenylendiamin erhalten wird, |
wobei das nicht-aromatische Lösungsmittel ausgewählt wird aus Methanol, Ethanol, Isopropanol, n-Propanol, Ethylformiat, Methylacetat, Ethylacetat, Methylpropionat, Ethylpropionat, CHCl₃, CCl₄, THF, 1,4-Dioxan, oder Kombinationen davon,
wobei das aromatische Lösungsmittel ausgewählt wird aus Toluol, Xylol, Anisol, Kresol, und Kombinationen davon.

6. Verfahren nach Anspruch 5, wobei in Schritt K die Menge an Aktivkohle 3-20 Gewichtsteile pro 100 Gewichtsteile 2-Methoxymethyl-p-phenylendiamin-Ausgangsmaterial beträgt, insbesondere wobei die Menge an Aktivkohle in Schritt K 5-10 Gewichtsteile pro 100 Gewichtsteile 2-Methoxymethyl-p-phenylendiamin-Ausgangsmaterial beträgt.

7. Verfahren nach Anspruch 5 oder 6, wobei in Schritt K die Menge an nicht-aromatischem Lösungsmittel innerhalb des Bereichs von 700-1200 Volumenteilen oder 800-1100 Volumenteilen liegt, insbesondere 900-1000 Volumenteile, wie etwa 930-980 Volumenteile.

8. Verfahren nach einem der Ansprüche 5-7, wobei die dritte Zieltemperatur innerhalb des Bereichs von 50-100°C liegt, insbesondere innerhalb des Bereichs von 70-90°C, beispielsweise innerhalb des Bereichs von 75-85°C, wie etwa innerhalb des Bereichs von 78-82°C, und/oder wobei die vierte Zieltemperatur bei einem Druck innerhalb des Bereichs von 120-280 mbar innerhalb des Bereichs von 35-48°C liegt, und/oder wobei die fünfte Zieltemperatur eine Temperatur unter 6,0°C ist, insbesondere innerhalb des Bereichs von 0,0-4,0°C, beispielsweise 0,0-3,0°C, wie etwa 0,0-2,5°C.

9. Verfahren nach einem der Ansprüche 5-8, des Weiteren umfassend Schritt O:
| | |
|---|---|
| O | Einstellen des Filtrats auf ein Verhältnis von 1000-1500 Volumenteilen, insbesondere von 1100-1300 Volumenteilen, wie etwa von 1150-1250 Volumenteilen nicht-aromatisches Lösungsmittel pro 100 Gewichtsteile des 2-Methoxymethyl-p-phenylendiamin-Ausgangsmaterials. |

10. Verfahren nach einem der Ansprüche 5-9, wobei in Schritt Q das Volumenverhältnis von pro Zeiteinheit zugegebenem aromatischem Lösungsmittel zu pro Zeiteinheit entferntem organischen Lösungsmittel innerhalb des Bereichs von 3,0-1,0 liegt, insbesondere innerhalb des Bereichs von 2,5-1,5, beispielsweise etwa 2,0 beträgt.

11. Verfahren nach einem der Ansprüche 5-10, wobei das in Schritt R erhaltene Verhältnis 500-600 Volumenteile organisches Lösungsmittel pro 100 Gewichtsteile des 2-Methoxymethyl-p-phenylendiamin-Ausgangsmaterials beträgt, insbesondere wobei das Verhältnis 500-550 Volumenteile organisches Lösungsmittel pro 100 Gewichtsteile des 2-Methoxymethyl-p-phenylendiamin-Ausgangsmaterials beträgt, beispielsweise wobei das Verhältnis 500-520 Volumenteile organisches Lösungsmittel pro 100 Gewichtsteile des 2-Methoxymethyl-p-phenylendiamin-Ausgangsmaterials beträgt.

12. Verfahren nach einem der Ansprüche 5-11, wobei in den Schritten P-R die Temperatur innerhalb der vierten Zieltemperatur gehalten wird und die Destillationsgeschwindigkeit durch Einstellen des Drucks geregelt wird.

13. Verfahren nach einem der Ansprüche 5-12, wobei das nicht-aromatische Lösungsmittel Ethylacetat ist und/oder wobei das aromatische Lösungsmittel Toluol ist.

14. Verfahren nach einem der Ansprüche 2-13, wobei jeder der Schritte B-H und K-U unter feuchtigkeitsfreien Bedingungen in einer inerten Atmosphäre durchgeführt wird.

15. Verfahren nach einem der Ansprüche 2-14, des Weiteren umfassend Schritt A und/oder Schritt Z:
| | |
|---|---|
| A | Bestimmen der UV-Absorption einer wässrigen Lösung des Ausgangsmaterials 2-Methoxymethyl-p-phenylendiamin bei 525 nm, |
| Z | Bestimmen der UV-Absorption einer wässrigen Lösung des erhaltenen 2-Methoxymethyl-p-phenylendiamins bei 525 nm. |

## Revendications

1. Procédé d'évaluation de la qualité de la 2-méthoxyméthyl-p-phénylènediamine, comprenant :
i. la détermination à 525 nm de l'absorbance UV d'une solution aqueuse de 2-méthoxyméthyl-p-phénylènediamine,
ii. la classification de la qualité de la 2-méthoxyméthyl-p-phénylènediamine sur la base de l'absorbance UV obtenue,
éventuellement dans lequel la solution aqueuse de l'étape (i) comprend un antioxydant.

2. Procédé de conversion d'une matière de départ 2-méthoxyméthyl-p-phénylènediamine colorée présentant un degré d'impureté inacceptable pour des applications cosmétiques en 2-méthoxyméthyl-p-phénylènediamine répondant aux exigences des applications cosmétiques, la matière de départ colorée étant détériorée par oxydation de surface lors du stockage pendant plusieurs semaines ou mois, le procédé comprenant les étapes indiquées dans un ordre alphabétique :
B préparation d'un mélange de 100 parties en poids de la matière de départ 2-méthoxyméthyl-p-phénylènediamine détériorée colorée, de charbon actif, et d'une quantité de solvant aromatique suffisante pour dissoudre la 2-méthoxyméthyl-p-phénylènediamine,
C chauffage du mélange de l'étape B à une première température cible,
E élimination du charbon actif par filtration à chaud, ce qui donne ainsi un filtrat,
F refroidissement du filtrat à une deuxième température cible,
H filtration à froid du filtrat, ce qui donne ainsi un résidu de 2-méthoxyméthyl-p-phénylènediamine recristallisée,
dans lequel le solvant aromatique est choisi parmi le toluène, le xylène, l'anisole, le crésol, et leurs combinaisons.

3. Procédé selon la revendication 2, dans lequel, à l'étape B, la quantité de charbon actif est de 3 à 20 parties en poids pour 100 parties en poids de matière de départ 2-méthoxyméthyl-p-phénylènediamine, en particulier dans lequel la quantité de charbon actif à l'étape B est de 5 à 10 parties en poids pour 100 parties en poids de matière de départ 2-méthoxyméthyl-p-phénylènediamine, éventuellement dans lequel, à l'étape B, la quantité de solvant aromatique est située dans la plage de 500 à 900 parties en volume, ou de 600 à 800 parties en volume, en particulier de 650 à 750 parties en volume, telle que de 680 à 720 parties en volume.

4. Procédé selon la revendication 2 ou 3, dans lequel la première température cible est située dans la plage de 60 à 100°C, en particulier dans la plage de 70 à 90°C, par exemple dans la plage de 75 à 85°C, telle que dans la plage de 78 à 82°C, et/ou dans lequel la deuxième température cible est inférieure à 8°C, par exemple dans la plage de 0,0 à 5,0°C, en particulier 0,0 à 4,0°C, telle que 0,0 à 3,0°C.

5. Procédé de conversion d'une matière de départ de 2-méthoxyméthyl-p-phénylènediamine colorée présentant un degré d'impureté inacceptable pour des applications cosmétiques en 2-méthoxyméthyl-p-phénylènediamine répondant aux exigences des applications cosmétiques, la matière de départ colorée étant détériorée par oxydation de surface lors du stockage pendant plusieurs semaines ou mois, le procédé comprenant les étapes indiquées dans un ordre alphabétique :
K préparation d'un mélange de 100 parties en poids de la matière de départ 2-méthoxyméthyl-p-phénylènediamine détériorée colorée, de charbon actif, et d'une quantité de solvant non aromatique suffisante pour dissoudre la 2-méthoxyméthyl-p-phénylènediamine,
L chauffage du mélange de l'étape K à une troisième température cible,
N élimination du charbon actif par filtration, ce qui donne ainsi un filtrat,
P amenée le filtrat à une quatrième température cible, et sous pression réduite réduction de la quantité de solvant non aromatique dans le filtrat par distillation,
Q ajout de solvant aromatique au filtrat,
R distillation du filtrat sous pression réduite à la quatrième température cible jusqu'à obtention d'un rapport de 700 parties en volume ou moins de solvant organique pour 100 parties en poids de la matière de départ 2-méthoxyméthyl-p-phénylènediamine,
S augmentation de la pression à la pression ambiante, et refroidissement du filtrat à une cinquième température cible,
U filtration du filtrat à la cinquième température cible, ce qui donne ainsi un résidu de 2-méthoxyméthyl-p-phénylènediamine recristallisée,
dans lequel le solvant non aromatique est choisi parmi le méthanol, l'éthanol, l'iso-propanol, le n-propanol, le formiate d'éthyle, l'acétate de méthyle, l'acétate d'éthyle, le propionate de méthyle, le propionate d'éthyle, CHCl₃, CCl₄, THF, le 1,4-dioxane, ou leurs combinaisons,
dans lequel le solvant aromatique est choisi parmi le toluène, le xylène, l'anisole, le crésol, et leurs combinaisons.

6. Procédé selon la revendication 5, dans lequel, à l'étape K, la quantité de charbon actif est de 3 à 20 parties en poids pour 100 parties en poids de matière de départ 2-méthoxyméthyl-p-phénylènediamine, en particulier dans lequel la quantité de charbon actif à l'étape K est de 5 à 10 parties en poids pour 100 parties en poids de matière de départ 2-méthoxyméthyl-p-phénylènediamine.

7. Procédé selon la revendication 5 ou 6, dans lequel, à l'étape K, la quantité de solvant non aromatique est située dans la plage de 700 à 1200 parties en volume, ou de 800 à 1100 parties en volume, en particulier de 900 à 1000 parties en volume, telle que de 930 à 980 parties en volume.

8. Procédé selon l'une quelconque des revendications 5 à 7, dans lequel la troisième température cible est située dans la plage de 50 à 100°C, en particulier dans la plage de 70 à 90°C, par exemple dans la plage de 75 à 85°C, telle que dans la plage de 78 à 82°C, et/ou dans lequel la quatrième température cible est située dans la plage de 35 à 48°C à une pression située dans la plage de 120 à 280 mbar, et/ou dans lequel la cinquième température cible est une température inférieure à 6,0°C, en particulier dans la plage de 0,0 à 4,0°C, par exemple de 0,0 à 3,0°C, telle que de 0,0 à 2,5°C.

9. Procédé selon l'une quelconque des revendications 5 à 8, comprenant en outre l'étape O :
O ajustement du filtrat à un rapport de 1000 à 1500 parties en volume, en particulier de 1100 à 1300 parties en volume, tel que de 1150 à 1250 parties en volume de solvant non aromatique pour 100 parties en poids de matière de départ 2-méthoxyméthyl-p-phénylènediamine.

10. Procédé selon l'une quelconque des revendications 5 à 9, dans lequel, à l'étape Q, le rapport en volume de solvant aromatique ajouté par unité de temps au solvant organique éliminé par unité de temps est situé dans la plage de 3,0 à 1,0, en particulier dans la plage de 2,5 à 1,5, tel qu'environ 2,0.

11. Procédé selon l'une quelconque des revendications 5 à 10, dans lequel le rapport obtenu à l'étape R est de 500 à 600 parties en volume de solvant organique pour 100 parties en poids de la matière de départ 2-méthoxyméthyl-p-phénylènediamine, en particulier dans lequel le rapport est de 500 à 550 parties en volume de solvant organique pour 100 parties en poids de la matière de départ 2-méthoxyméthyl-p-phénylènediamine, par exemple dans lequel le rapport est de 500 à 520 parties en volume de solvant organique pour 100 parties en poids de la matière de départ 2-méthoxyméthyl-p-phénylènediamine.

12. Procédé selon l'une quelconque des revendications 5 à 11, dans lequel, aux étapes P à R, la température est maintenue à la quatrième température cible, et la vitesse de distillation est contrôlée par ajustement de la pression.

13. Procédé selon l'une quelconque des revendications 5 à 12, dans lequel le solvant non aromatique est l'acétate d'éthyle, et/ou dans lequel le solvant aromatique est le toluène.

14. Procédé selon l'une quelconque des revendications 2 à 13, dans lequel chacune des étapes B à H et K à U est réalisée dans des conditions exemptes d'humidité sous atmosphère inerte.

15. Procédé selon l'une quelconque des revendications 2 à 14, comprenant en outre l'étape A et/ou l'étape Z :
A détermination à 525 nm de l'absorbance UV d'une solution aqueuse de la matière de départ 2-méthoxyméthyl-p-phénylènediamine,
Z détermination à 525 nm de l'absorbance UV d'une solution aqueuse de la 2-méthoxyméthyl-p-phénylènediamine obtenue.
